# EUROPÄISCHE PATENTSCHRIFT

(11) **EP 3 037 400 B1**
(45) Veröffentlichungstag und Bekanntmachung des Hinweises auf die Patenterteilung: **01.08.2018**
(21) Anmeldenummer: 15201166.4
(22) Anmeldetag: 18.12.2015
(51) Int. Cl.: C07C 29/141, B01J 23/755

(54) **CHROMFREIE HYDRIERUNG VON HYDROFORMYLIERUNGSGEMISCHEN**
Chromium-free hydrogenation of hydroformylation mixtures
Hydrogénation sans chrome de mélanges d'hydroformylation

(30) Priorität: 23.12.2014 EP 14199938
(43) Veröffentlichungstag der Anmeldung: 29.06.2016
(73) Patentinhaber: Evonik Degussa GmbH, 45128 Essen (DE)
(72) Erfinder: Klasovsky, Florian, 45721 Haltern am See (DE); Franke, Robert, 45772 Marl (DE); Becker, Marc, 44359 Dortmund (DE); Quandt, Thomas, 45772 Marl (DE); Geilen, Frank, 45721 Haltern am See (DE)

(56) Entgegenhaltungen:
- DE-A1- 3 737 277
- DE-A1-102008 007 080

## Beschreibung

Die Erfindung betrifft ein Verfahren zur Herstellung von Alkoholen durch Hydrierung von Aldehyden, bei welchem ein Einsatzgemisch enthaltend mindestens einen Aldehyd sowie mindestens eine Begleitkomponente in Gegenwart von Wasserstoff mit einem heterogenen Katalysator in Kontakt gebracht wird, wodurch ein Produktgemisch erhalten wird, welches zumindest den hydrierten Aldehyd entsprechenden Alkohol sowie zumindest ein Nebenprodukt enthält, wobei der Katalysator ein Trägermaterial sowie darauf aufgebracht Nickel und Kupfer umfasst.

Durch Abspaltung von Wasserstoff (Dehydrierung) von einem Alkohol entsteht ein Aldehyd. Umgekehrt lassen sich Alkohole durch Hydrierung (Anlagerung von Wasserstoff) von Aldehyden herstellen.

Die Hydrierung im Allgemeinen ist eine in der industriellen Technik sehr häufig durchgeführte Reaktion. Aber auch speziell die Hydrierung von Aldehyden wird großindustriell praktiziert, nämlich bei der Herstellung von so genannten Oxo-Alkoholen.

Oxo-Alkohole sind Alkohole, die im Wege der Hydroformylierung (Oxo-Reaktion) hergestellt werden. Bei der Hydroformylierung wird ein Olefin (Alken) mit Synthesegas (das ist eine Mischung aus Kohlenmonoxid und Wasserstoff) zu einem Aldehyd umgesetzt. Durch anschließende Hydrierung wird der eigentliche Oxo-Alkohol erhalten. Oxo-Alkohole dienen als Zwischenprodukte für die Herstellung von Tensiden bzw. Weichmachern für Kunststoff. Weltweit werden jährlich mehrere Millionen Tonnen Oxo-Alkohole hergestellt.

Da die Hydrierung der durch die Hydroformylierung erhaltenen Aldehyde ein notwendiger Schritt bei der Herstellung von Oxo-Alkoholen ist, beschäftigt sich die vorliegende Erfindung mit einem großindustriell relevanten Prozess.

In der industriellen Praxis werden Oxo-Aldehyde in der Regel in der Flüssigphase an heterogenen Festbett-Katalysatoren hydriert. Aufgrund der großen Durchsatzmengen kommt dem Katalysator die entscheidende Bedeutung für den Prozess zu, da er die Reaktionsgeschwindigkeit und auch die Selektivität der Hydrierung bestimmt. Die Auswahl eines geeigneten Katalysators ist nicht trivial, da die zu hydrierenden Aldehyde niemals rein vorkommen, sondern als ein Gemisch von strukturisomeren Aldehyden, welches stets von einer großen Anzahl störender Begleitkomponenten begleitet wird, die zum einen in der Hydrierung unerwünschte Nebenreaktionen hervorrufen und zum anderen den Hydrierkatalysator schädigen. Da die Zusammensetzung des zu hydrierenden Aldehyde enthaltenden Einsatzgemisches durch die vorgelagerte Hydroformylierung bestimmt wird, ist der Hydrierkatalysator auf die jeweilige Hydroformylierung genau anzupassen.

Bei der Hydrierung von Oxo-Aldehyden haben sich solche Katalysatoren bewährt, die ein Trägermaterial umfassen, auf welchem als aktive Komponenten Kupfer, Chrom und Nickel aufgebracht sind.

Ein entsprechender Katalysator ist in DE19842370A1 offenbart. Er umfasst Kupfer und Nickel jeweils in einem Konzentrationsbereich von 0.3 bis 15 Gew.-% und Chrom zu einem Gewichtsanteil von 0.05 Gew.-% bis 3.5 Gew.-%. Als Trägermaterial wird poröses Siliciumdioxid oder Aluminiumoxid verwendet.

Aus EP1219584B1 geht hervor, dass ein solcher Katalysator auch mit der Begleitkomponente Wasser zurechtkommt, die insbesondere bei der Co-katalysierten Hydroformylierung zu erwarten ist. Wasser ist kritisch, da es die Oberflächenstruktur des Katalysators nachhaltig negativ beeinflussen kann, indem es beispielsweise die spezifische Oberfläche reduziert. Aus diesem Grund sind die aus der Cobalt-katalysierten Hydroformylierung stammenden Aldehydgemische besonders anspruchsvoll zu hydrieren.

Eine Weiterentwicklung dieses Ni/Cu/Cr-Katalysators besteht darin, dass dem Trägermaterial Barium zugegeben wird (EP2180947B1).

Die WO2009/146988A1 beschäftigt sich mit der zweistufigen Hydrierung von Oxo-Alkoholen an zwei unterschiedlichen Ni/Cu/Cr-Katalysatoren.

Die DE 10 2008 007 080 A1 beschreibt ein mehrstufiges Verfahren bei der nach vorheriger Hydroformylierung aus den erhaltenen Aldehyden Alkohole mittels Hydrierung hergestellt werden. Dazu werden verschiedene Katalysatorvarianten vorgeschlagen, von denen die Katalysatoren, die eine Menge von 0,05 bis 3,5 Massen-% Chrom enthalten bevorzugt sind.

Wenngleich sich die Nickel/Kupfer/Chrom-Katalysatoren in der industriell praktizierten Hydrierung von Oxo-Aldehyden bewährt haben, bestand doch das Bedürfnis nach einer Alternative. Grund dafür ist das enthaltende Chrom.

Gemäß Annex XIV der REACH-Verordnung dürfen chromhaltige Substanzen wie die vorbeschriebenen Katalysatoren in der Europäischen Union nur noch nach Autorisierung durch die Kommission verwendet werden. Die Erteilung der Autorisierung ist mit hohem Aufwand und Kosten verbunden; zudem kann nicht a priori mit der Erteilung der Autorisierung gerechnet werden. Die Anmeldeprozedur muss darüber hinaus alle fünf Jahre wiederholt werden.

Grund für diese strengen Auflagen ist die unbestrittene Kanzerogenität der im Katalysator enthaltenden Chrom(IV)-Verbindungen. Diese sind zum einen dann relevant, wenn Hydrierkatalysator nach Desaktivierung entsorgt werden muss und zum anderen wenn er durch Imprägnierung mit Alkalichromaten oder Alkalidichromaten neu hergestellt wird.

Aus gesundheitlichen und wirtschaftlichen Gründen besteht somit ein großer Bedarf an einer chromfreien Alternative zur Hydrierung von Oxo-Aldehyden.

Chromfreie Hydrier-Katalysatoren sind in EP1749572A1 offenbart. Als Trägermaterial wird poröses Aluminiumoxid verwendet und als hydrieraktive Komponenten Nickel oder Cobalt. Aus den Beispielen geht hervor, dass ein Ni/Al2O3 oder ein Co/Al2O3-System zur Hydrierung von Oxo-Aldehyden geeignet ist; die Eigenschaften und Leistungsfähigkeit dieser Systeme wurden jedoch nicht untersucht.

Der Nachteil der in EP1749572A1 gezeigten Cobalt-Katalysatoren besteht darin, dass sie auf jeden Fall bei höheren Temperaturen von 350 °C bis 450 °C reduziert werden müssen. Dies erfolgt üblicherweise nicht in-situ im Reaktor, da die Reaktoren für die Aldehydhydrierung lediglich bis auf eine Temperatur von etwa 200 °C ausgelegt sind. Mithin müssen die Cobalt-Katalysatoren ex situ reduziert und sodann unter Schutzatmosphäre in den Hydrier-Reaktor eingebaut werden. Dies ist sehr aufwendig. Außerdem ist Cobalt ein vergleichsweise teures Material.

Bei den Nickelkatalysatoren könnten ca. 200 °C für eine in-situ Reaktion gerade ausreichen. Allerdings erwähnt EP1749572A1, dass das Ni/Al2O3-System die Weiterreaktion begünstigt und die Reaktion daher sehr schnell beendet werden muss. Das ist im großtechnischen Einsatz nicht immer einfach zu bewerkstelligen.

Die in EP1749572A1 gezeigten chromfreien Hydrier-Katalysatoren weisen insgesamt so viele Nachteile auf, dass sie keine echte Alternative zu den klassischen Ni/Cu/Cr-Systemen darstellen.

Aus der DE3737277C2 ist ein chromfreier Katalysator zum Hydrieren von Aldehyden bekannt,
welcher auf Kupfer/Zinkoxiden basiert. Als weitere hydrieraktive Metalle sind Kalium, Nickel und/oder Cobalt und zusätzlich ein Alkalimetall enthalten. Bei diesem System handelt es sich um einen so genannten Vollkatalysator, der ausschließlich aus den hydrieraktiven Materialien besteht. Solche Vollkatalysatoren sind sehr aufwändig herzustellen und deswegen für den industriellen Einsatz zu kostspielig.

Nach alldem ist es bisher nicht gelungen, einen chromfreien Katalysator zu finden, der sich für die Hydrierung von Hydroformylierungsgemischen im großtechnischen Maßstab eignet und der sich einfach herstellen lässt.

Die Aufgabe der Erfindung besteht somit darin, ein Verfahren mit einem solchen Katalysator bereitzustellen. Insbesondere soll derselbe Katalysator geeignet sein, Aldehydgemische mit unterschiedlichen Kettenlängen zu hydrieren, insbesondere solche, die aus unterschiedlichen Hydroformylierungen stammen und auch Substanzen mit C=C-Doppelbindungen enthalten können.

Gelöst wurde diese Aufgabe überraschenderweise dadurch, dass das Chrom bei der Herstellung eines klassischen Cu/Ni/Cr-Systems weggelassen wird, sodass ein Katalysator erhalten wird, auf dessen Trägermaterial lediglich Kupfer und Nickel als hydrieraktive Komponenten vorkommen, nicht aber Chrom.

Dieser Befund ist deswegen überraschend, da in der Vergangenheit stets davon ausgegangen wurde, dass Chrom eine notwendige Komponente für derartige Hydrieraufgaben darstellt.

Tatsächlich belegen Langzeitversuche, dass ein geringer Chromanteil von 0.05 Gew.-%bis 3.5 Gew.-% zwar durchaus im Anfahrbereich Vorteile bietet, langfristig aber die Performance von Chromfreien und Chromhaltigen Cu/Ni-Systemen in etwa gleich ist. Deswegen bestehen nunmehr keine Bedenken mehr gegen die Verwendung von chromfreien Cu/Ni-Systemen in der Hydrierung von Hydroformylierungsgemischen.

Gegenstand der Erfindung ist mithin ein Verfahren zur Herstellung von Alkoholen durch Hydrierung von Aldehyden, bei welchem ein Einsatzgemisch enthaltend mindestens einen Aldehyd sowie mindestens eine Begleitkomponente in Gegenwart von Wasserstoff mit einem heterogenen Katalysator in Kontakt gebracht wird, wodurch ein Produktgemisch erhalten wird, welches zumindest den dem hydrierten Aldehyd entsprechenden Alkohol sowie zumindest ein Nebenprodukt enthält, wobei der Katalysator ein Trägermaterial sowie darauf aufgebracht Nickel und Kupfer umfasst, wobei der Katalysator in aktivierter Form die folgende, sich zu 100 Gew.-% ergänzende Zusammensetzung aufweist:
- Trägermaterial:: von 85 Gew.-% bis 95 Gew.-%, vorzugsweise von 88 Gew.-% bis 92 Gew.-%;
- Kupfer:: von 5.3 Gew.-% bis 8.4 Gew.-%, vorzugsweise von 6.5 Gew.-% bis 7.0 Gew.-%;
- Nickel:: von 2.2 Gew.-% bis 3.9 Gew.-%, vorzugsweise von 2.8 Gew.-% bis 3.3 Gew.-%;
- Chrom:: weniger als 50 Gew.-ppm, vorzugsweise weniger als 5 Gew.-ppm;
- Sonstige:: weniger als 1 Gew.-%,
und wobei es sich bei dem Trägermaterial um Aluminiumoxid oder um Silicumdioxid oder um ein Gemisch aus Aluminiumoxid und Siliciumdioxid handelt.

Ein solcher Katalysator kann als chromfrei bezeichnet werden "Frei von Chrom" bedeutet in diesem Zusammenhang, dass bei der Herstellung des Katalysators aktiv kein Chrom verwendet wurde, insbesondere, dass das Trägermaterial nicht mit chromhaltigen Substanzen imprägniert worden ist. Als chromhaltige Substanzen sind insbesondere Verbindungen zu nennen, die Cr-III und Cr-VI enthalten.

Aufgrund der heutigen Analysemöglichkeiten lässt sich es nicht ausschließen, dass sich Spuren von Chrom im erfindungsgemäßen Katalysator nachweisen lassen. Diese könnten beispielsweise aus dem Stahl der Apparaturen stammen, mit denen der Katalysator bei seiner Herstellung, seiner Lagerung, seines Transports, seines Einbaus oder seines Einsatzes in Kontakt kommt. Dies ist aber kein gewollter Chromgehalt.

Soweit erfindungsgemäße Chromfreiheit mit einem Zahlenwert definiert werden soll, so wird ein Katalysator, der Bezogen auf sein Gesamtgewicht weniger als 50 Gew.-ppm Chrom enthält, als "chromfrei" im Sinne dieser Erfindung angesehen. Vorzugsweise enthält der Katalysator sogar weniger als 5 Gew.-ppm Chrom. Die Maßeinheit ppm wird hier als 10⁻⁶ verstanden. Die angegebenen Grenzwerte für Chrom beziehen sich insbesondere auf den Gesamtgehalt von Substanzen, die Cr-III und Cr-VI enthalten.

Im Vergleich zu dem aus DE19842370A1 bekannten Katalysator, der mindestens 0.05 Gew.-% (entspricht 500 ppm) Chrom enthält, liegt der Chromgehalt eines erfindungsgemäßen Katalysators ein bis zwei Größenordnungen darunter.

Indem bei der Herstellung des Katalysators auf eine Imprägnierung des Trägermaterials mit Alkalichromaten oder Alkalidichromaten verzichtet wird, gelingt es, die geforderte Chromfreiheit zu gewährleisten.

Als Trägermaterial kommen grundsätzlich solche Substanzen in Betracht, die sich in der Hydrierung inert verhalten (also nicht mitreagieren) und befähigt sind, die katalytisch aktiven Substanzen Nickel und Kupfer zu tragen. Anders als ein Vollkatalysator enthält der hier beschriebene Trägerkatalysator folglich das inerte Trägermaterial, auf welchem das Aktivmaterial aufgebracht wird. Geeignete Trägermaterialien sind kommerziell verfügbar und lassen sich mit im industriellen Maßstab mit Hilfe bewährter Technologie mit den Aktivmaterialen belegen.

Bei dem Trägermaterial handelt es sich um Aluminiumoxid oder um Silicumdioxid oder es stellt ein Gemisch aus Aluminiumoxid und Siliciumdioxid (Silica/Alumina) dar.

Das Trägermaterial sollte porös sein. Das spezifische Porenvolumen des Trägermaterials sollte zwischen 0.5 ml/g bis 0.9 ml/g betragen. Der Wert des spezifischen Porenvolumens wird nach der Cyclohexan-Immersionsmethode bestimmt. Dabei wird eine Probe des Trägermaterials in ein Gefäß gelegt und das Gefäß evakuiert. Die Probe in dem luftfreien Gefäß wird gewogen. Sodann wird das Gefäß mit einer vorher volumetrisch gemessenen Menge Cyclohexan geflutet, sodass die Probe darin getränkt ist. Man lässt das Cyclohexan in die Probe eindringen. Sodann wird das Cyclohexan abgeschüttet und die abgeschüttete Menge volumetrisch gemessen. Die Differenz zu der eingefüllten Menge Cyclohexan entspricht der in die Poren eingedrungenen Menge, also dem Porenvolumen. Dieses Porenvolumen wird dann auf das Gewicht der Probe bezogen. Auf diese Weise erhält man das spezifische Porenvolumen des Trägers.

Die spezifische Oberfläche des Trägermaterials (BET-Oberfläche) sollte zwischen 240 m²/g bis 280 m²/g betragen. Die spezifische Oberfläche wird nach der ISO-Methode 9277 bestimmt.

Die Hydrierung sollte bei einem Druck zwischen 15*10⁵ Pa und 25*10⁵ Pa und bei einer Temperatur zwischen 140°C und 180°C durchgeführt werden, wobei Druck und Temperatur so zu wählen sind, dass Einsatzgemisch und Produktgemisch in einer flüssigen Phase vorliegen. Dies steigert die Prozessintensität.

Der Wasserstoff sollte überstöchiometrisch in der Hydrierung gegenwärtig sein um eine vollständige Hydrierung zu ermöglichen. Allerdings solle die Konzentration des Wasserstoffes so gewählt werden, dass zumindest ein Teil des Wasserstoffs in der flüssigen Phase gelöst vorliegt. Dadurch wird die Strömungsdynamik nicht so stark durch Gasblasen negativ beeinflusst.

Erfindungsgemäß werden Oxo-Aldehyde zu den entsprechenden Alkoholen hydriert. Mithin stammt das Einsatzgemisch aus einer Hydroformylierung und weist als solches mehrere Aldehyde mit derselben Anzahl n an Kohlenstoffatomen sowie entsprechende Alkohole und Hochsieder auf, wobei es sich bei n um eine natürliche Zahl zwischen drei und achtzehn handelt.

Vorzugsweise weist das Einsatzgemisch eine der folgenden Spezifikationen A bis E auf, wobei sich die spezifizierten Zusammensetzungen jeweils zu 100 Gew.-% ergänzen:

### Spezifikation A:

| | |
|---|---|
| Gesamtanteil der Aldehyde mit fünf Kohlenstoffatomen: | 80 Gew.-% bis 100 Gew.-%; |
| Gesamtanteil der Alkohole mit fünf Kohlenstoffatomen: | 0 Gew.-% bis 1 Gew.-%; |
| Gesamtanteil an anderen Kohlenwasserstoffen: | 2 Gew.-% bis 20 Gew.-%. |

### Spezifikation B:

| | |
|---|---|
| Gesamtanteil der Aldehyde mit neun Kohlenstoffatomen: | 25 Gew.-% bis 75 Gew.-%; |
| Gesamtanteil der Alkohole mit neun Kohlenstoffatomen: | 10 Gew.-% bis 55 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 3 Gew.-%. |

### Spezifikation C:

| | |
|---|---|
| Gesamtanteil der Aldehyde mit neun Kohlenstoffatomen: | 15 Gew.-% bis 65 Gew.-%; |
| Gesamtanteil der Alkohole mit neun Kohlenstoffatomen: | 20 Gew.-% bis 65 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 1 Gew.-%. |

### Spezifikation D:

| | |
|---|---|
| Gesamtanteil der Aldehyde mit zehn Kohlenstoffatomen: | 50 Gew.-% bis 100 Gew.-%; |
| Gesamtanteil der Alkohole mit zehn Kohlenstoffatomen: | 0 Gew.-% bis 40 Gew.-%; |
| Gesamtanteil weitere Kohlenwasserstoffe: | 0 Gew.-% bis 5 Gew.-%; |
| Anteil an Wasser: | 0.5 Gew.-% bis 5 Gew.-%. |

### Spezifikation E:

| | |
|---|---|
| Gesamtanteil der Aldehyde mit dreizehn Kohlenstoffatomen: | 60 Gew.-% bis 85 Gew.-%; |
| Gesamtanteil der Alkohole mit dreizehn Kohlenstoffatomen: | 1 Gew.-% bis 20 Gew.-%; |
| Gesamtanteil weitere Kohlenwasserstoffe: | 10 Gew.-% bis 40 Gew.-%; |
| Anteil an Wasser: | 0.1 Gew.-% bis 1 Gew.-%. |

Ein besonderer Vorteil des gefundenen Katalysators besteht darin, dass er auch für die Mischhydrierung geeignet ist, also ein Einsatzgemisch verarbeiten kann, welches aus zwei oder mehr unterschiedlichen Hydroformylierungsausträgen zusammengesetzt ist. So ist es an Standorten, wo mindestens zwei Hydroformylierungen parallel betrieben werden, möglich, die Hydroformylierungsgemische beider Oxo-Anlagen zusammenzufassen und in einem Hydrierprozess an dem hier gefundenen Katalysator zu hydrieren. Dies spart Investitionskosten für die Hydrieranlage. Wenn die erste Oxo-Anlage etwa Aldehyde mit n Kohlenstoffatomen produziert und die zweite Oxo-Anlage Aldehyde mit m Kohlenstoffatomen, dann enthält das vereinigte Einsatzgemisch für die Hydrierung mehrere Aldehyde mit derselben Anzahl n an Kohlenstoffatomen und mehrere Aldehyde mit derselben Anzahl m an Kohlenstoffatomen, sowie jeweils entsprechende Alkohole und Hochsieder, wobei es sich bei n und m um unterschiedliche natürliche Zahlen zwischen drei und achtzehn handelt. Die Hydrierung eines solchen, aus mindestens zwei unterschiedlichen Hydroformylierungen stammenden Einsatzgemisches ist besonders bevorzugt.

Der erfindungsgemäß eingesetzte, chromfreie Katalysator wird wie folgt hergestellt:
a) Bereitstellen eines Trägermaterials;
b) Imprägnieren des Trägermaterials mit einer Chromfeien Lösung aus Kupfer(II)hydroxid-carbonat, Nickelhydroxycarbonatpaste, Ammoniumcarbonat, Ammoniak und Wasser;
c) Trocknen des imprägnierten Trägermaterials im Luftstrom bei Temperaturen unter 100 °C;
d) Kalzinieren des getrockneten, imprägnierten Trägermaterials im Luftstrom bei Temperaturen unter 450 °C;
e) Aktivierung des kalzinierten, getrockneten, imprägnierten Trägermaterials durch Reduktion mit Wasserstoff unter Erhalt des aktiven Katalysators, wobei die Aktivierung in situ oder ex situ erfolgt.

Die Erfindung soll nun anhand von Beispielen näher erläutert werden.

### Beispiel 0

Ein geformter poröser Aluminiumoxidträger (Extrudate mit einem Durchmesser von etwa 1,2 mm, einer BET-Oberfläche von etwa 260 m²/g und einem Porenvolumen von etwa 0,7 ml/g) wird mit einer ammoniakalischen wässrigen Nickel- und Kupferverbindungen enthaltenden Lösung getränkt. Die wässrige Tränklösung wird aus Kupfer(II)hydroxid-carbonat, Nickelhydroxycarbonatpaste, Ammoniumcarbonat, wässriger Ammoniaklösung und Wasser erhalten. Die Tränkung kann durch verschiedene fachübliche Methoden erfolgen z.B. Sprühimprägnierung, Vakuumimprägnierung oder Tauchtränkung bei Normaldruck. Die Tränkung kann so erfolgen, dass die Menge der Lösung so gewählt wird, dass die Poren zum Teil oder vollständig mit Imprägnierlösung gefüllt werden oder der Träger in einem Überschuss an Lösung getränkt wird. Nach der Imprägnierung wird das Material im Luftstrom getrocknet (bei Temperaturen unter 100 °C). Die getrocknete Vorstufe wird anschließend im Luftstrom kalziniert (bei Temperaturen von 450 °C). Danach liegen Nickel und Kupfer in oxidischer Form in der Aluminiumoxidmatrix vor. Nach der Kalzination enthält die Katalysatorvorstufe 6,5 -7,0 % Kupfer, 2,8 - 3,3 % Nickel. Die Katalysatorvorstufe wird erst nach Aktivierung im Reaktor z.B. durch eine Reduktion der oxidischen Nickel- und Kupferverbindungen mit Wasserstoff in die eigentliche katalytisch aktive Form überführt.

### Beispiel 1

Ein Reaktionsaustrag aus der Cobalt-katalysierten Hydroformylierung von Dibuten wurde in einer Kreislaufapparatur bei 180°C und 25 bar absolut an 150 g Katalysator in der Flüssigphase kontinuierlich hydriert. Es wurden stündlich 0,90 l Edukt bei einem Kreislauf von 24 l/h durchgesetzt. Die Abgasmenge betrug 5 NL/h. Es wurde ein nach Beispiel 0 hergestellter, chromfreier Ni/Cu-Katalysator auf Al₂O₃-Trägermaterial verwendet. Die Zusammensetzung des verwendeten Einsatzgemisches ist in Tabelle 1 dargestellt.

**Tabelle 1: Zusammensetzung des Einsatzgemisches enthaltend C9-Aldehyde**

| Komponente | KW^{[a]} | ZL^{[b]} | AL^{[c]} | FOR^{[d]} | OL^{[e]} | Ether^{[f]} | Ester^{[g]} | Acetale^{[h]} | Wasser | Rest |
|---|---|---|---|---|---|---|---|---|---|---|
| Gehalt [Ma.%] | 5,5 | 0,1 | 69,2 | 0,3 | 21,7 | 0,0 | 0,8 | 1,6 | 0,7 | 0,0 |

| | | | | | | | | | | |
|---|---|---|---|---|---|---|---|---|---|---|
| [a] KW: Kohlenwasserstoffe; [b]: Zwischenlauf; [c]: C₉-Aldehyde; [d]: C₉-Formiate; [e]: C9-Alkohole; [f]: C₉/C₉-Ether; [g]: C₉/C₉-Ester; [h]: C₉/C₉/C₉-Acetale | | | | | | | | | | |

Die Zusammensetzung des Produktgemisches ist in Tabelle 2 wiedergegen.

**Tabelle 2: Zusammensetzung des Produktgemisches gemäß Beispiel 1 (Angaben in Ma.%)**

| t [h] | KW^{[a]} | ZL^{[b]} | AL^{[c]} | FOR^{[d]} | OL^{[e]} | Ether^{[f]} | Ester^{[g]} | Acetale^{[h]} | Rest |
|---|---|---|---|---|---|---|---|---|---|
| 18 | 5,3 | 0,1 | 2,0 | 0,0 | 89,5 | 0,0 | 0,7 | 2,3 | 0,0 |
| 25 | 5,3 | 0,1 | 2,1 | 0,0 | 90,0 | 0,0 | 0,6 | 1,8 | 0,1 |
| 42 | 5,2 | 0,1 | 2,2 | 0,0 | 89,8 | 0,0 | 0,6 | 1,9 | 0,1 |
| 49 | 5,3 | 0,1 | 2,3 | 0,0 | 89,6 | 0,0 | 0,6 | 1,9 | 0,1 |
| 66 | 5,3 | 0,1 | 2,3 | 0,0 | 89,4 | 0,0 | 0,6 | 2,0 | 0,1 |
| 73 | 5,2 | 0,1 | 2,4 | 0,0 | 89,5 | 0,0 | 0,6 | 2,0 | 0,1 |
| 89 | 5,4 | 0,1 | 2,6 | 0,0 | 89,1 | 0,0 | 0,6 | 2,1 | 0,1 |
| 97 | 5,4 | 0,1 | 2,5 | 0,0 | 89,0 | 0,0 | 0,6 | 2,2 | 0,1 |
| 162 | 5,4 | 0,1 | 2,8 | 0,0 | 88,6 | 0,0 | 0,6 | 2,4 | 0,1 |
| 169 | 5,4 | 0,1 | 2,8 | 0,0 | 88,6 | 0,0 | 0,6 | 2,3 | 0,1 |
| 186 | 5,4 | 0,1 | 2,7 | 0,0 | 89,3 | 0,0 | 0,6 | 1,9 | 0,1 |
| 193 | 5,4 | 0,1 | 2,8 | 0,0 | 89,1 | 0,0 | 0,6 | 1,9 | 0,1 |
| 210 | 5,4 | 0,1 | 2,8 | 0,0 | 89,0 | 0,0 | 0,6 | 2,0 | 0,1 |
| 217 | 5,4 | 0,1 | 2,9 | 0,0 | 89,0 | 0,0 | 0,6 | 1,9 | 0,1 |
| 234 | 5,3 | 0,1 | 3,0 | 0,0 | 88,8 | 0,0 | 0,6 | 2,0 | 0,1 |
| 241 | 5,3 | 0,1 | 3,0 | 0,0 | 88,9 | 0,0 | 0,6 | 2,0 | 0,1 |
| 258 | 5,4 | 0,1 | 3,1 | 0,0 | 88,7 | 0,0 | 0,6 | 2,0 | 0,1 |
| 265 | 5,4 | 0,1 | 3,0 | 0,0 | 88,6 | 0,0 | 0,6 | 2,1 | 0,1 |
| 330 | 5,4 | 0,1 | 3,3 | 0,0 | 88,3 | 0,0 | 0,6 | 2,2 | 0,0 |
| 337 | 5,4 | 0,1 | 3,2 | 0,0 | 88,9 | 0,0 | 0,6 | 1,8 | 0,0 |
| 354 | 5,3 | 0,1 | 3,2 | 0,0 | 89,3 | 0,0 | 0,6 | 1,5 | 0,0 |
| 361 | 5,3 | 0,1 | 3,2 | 0,0 | 89,3 | 0,0 | 0,6 | 1,4 | 0,0 |
| 378 | 5,3 | 0,1 | 3,2 | 0,0 | 89,3 | 0,0 | 0,6 | 1,5 | 0,0 |
| 385 | 5,3 | 0,1 | 3,3 | 0,0 | 89,2 | 0,0 | 0,6 | 1,4 | 0,0 |
| 402 | 5,3 | 0,1 | 3,4 | 0,0 | 89,1 | 0,0 | 0,6 | 1,5 | 0,0 |
| 409 | 5,3 | 0,1 | 3,5 | 0,0 | 89,1 | 0,0 | 0,6 | 1,4 | 0,0 |
| 426 | 5,3 | 0,1 | 3,6 | 0,0 | 88,9 | 0,0 | 0,6 | 1,4 | 0,0 |
| 433 | 5,3 | 0,1 | 3,5 | 0,0 | 88,9 | 0,0 | 0,6 | 1,5 | 0,0 |
| 498 | 5,4 | 0,1 | 3,9 | 0,0 | 88,5 | 0,0 | 0,6 | 1,6 | 0,0 |
| 505 | 5,4 | 0,1 | 3,8 | 0,0 | 88,6 | 0,0 | 0,6 | 1,5 | 0,0 |
| 522 | 5,4 | 0,1 | 3,9 | 0,0 | 88,3 | 0,0 | 0,6 | 1,6 | 0,1 |
| 529 | 5,6 | 0,1 | 3,9 | 0,0 | 88,2 | 0,0 | 0,6 | 1,6 | 0,0 |
| 546 | 5,4 | 0,1 | 4,0 | 0,0 | 88,2 | 0,0 | 0,6 | 1,6 | 0,0 |
| 553 | 5,4 | 0,1 | 4,0 | 0,0 | 88,2 | 0,0 | 0,6 | 1,6 | 0,0 |
| 570 | 5,4 | 0,1 | 4,1 | 0,0 | 88,0 | 0,0 | 0,6 | 1,8 | 0,0 |
| 577 | 5,5 | 0,1 | 4,1 | 0,0 | 87,8 | 0,0 | 0,6 | 1,8 | 0,0 |
| 594 | 5,4 | 0,1 | 4,2 | 0,0 | 87,9 | 0,0 | 0,6 | 1,8 | 0,0 |
| 601 | 5,4 | 0,1 | 4,2 | 0,0 | 87,8 | 0,0 | 0,6 | 1,8 | 0,0 |
| 666 | 5,4 | 0,1 | 4,4 | 0,0 | 87,6 | 0,0 | 0,6 | 1,9 | 0,0 |
| 673 | 5,4 | 0,1 | 4,4 | 0,0 | 87,3 | 0,0 | 0,6 | 2,2 | 0,0 |
| 690 | 5,3 | 0,0 | 4,5 | 0,0 | 87,6 | 0,0 | 0,6 | 1,9 | 0,0 |
| 697 | 5,4 | 0,0 | 4,5 | 0,0 | 87,5 | 0,0 | 0,6 | 1,9 | 0,1 |
| 714 | 5,3 | 0,0 | 4,6 | 0,0 | 87,5 | 0,0 | 0,6 | 1,9 | 0,0 |

| | | | | | | | | | |
|---|---|---|---|---|---|---|---|---|---|
| [a] KW: Kohlenwasserstoffe; [b]: Zwischenlauf; [c]: C₉-Aldehyde; [d]: C₉-Formiate; [e]: C9-Alkohole; [f]: C₉/C₉-Ether; [g]: C₉/C₉-Ester; [h]: C₉/C₉/C₉-Acetale | | | | | | | | | |

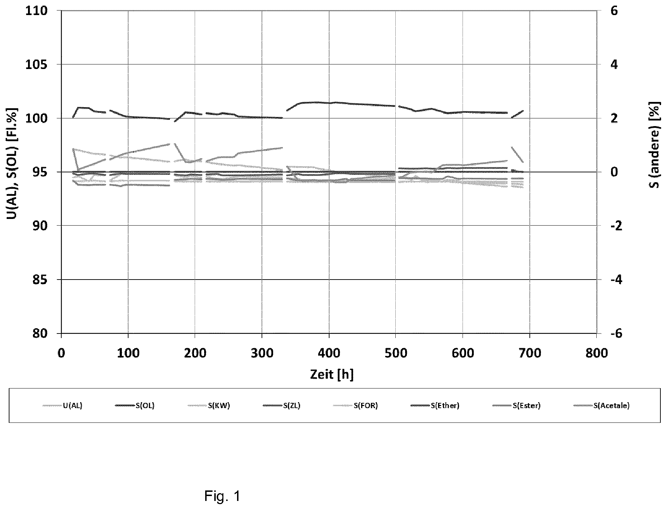

Ein zugehöriges Umsatz-Selektivitäts-Diagramm ist in Figur 1 dargestellt.

### Beispiel 2

Es wurde dasselbe Einsatzgemisch und dieselbe Apparatur wie in Beispiel 1 verwendet. Es wurde ein erfindungsgemäß chromfreier Katalysator verwendet.

Die Zusammensetzung des erhaltenen Produktgemisches ist in Tabelle 3 wiedergegen:

**Tabelle 3: Zusammensetzung des Produktgemisches gemäß Beispiel 2**

| t [h] | KW^{[a]} | ZL^{[b]} | AL^{[c]} | FOR^{[d]} | OL^{[e]} | Ether^{[f]} | Ester^{[g]} | Acetale^{[h]} | Rest |
|---|---|---|---|---|---|---|---|---|---|
| 18 | 5,8 | 0,0 | 2,2 | 0,5 | 89,1 | 0,0 | 0,2 | 1,8 | 0,3 |
| 25 | 5,8 | 0,0 | 2,4 | 0,4 | 88,4 | 0,0 | 0,7 | 2,0 | 0,2 |
| 41 | 5,8 | 0,0 | 2,5 | 0,5 | 88,1 | 0,0 | 0,6 | 2,2 | 0,3 |
| 49 | 6,2 | 0,0 | 2,6 | 0,6 | 87,5 | 0,0 | 0,5 | 2,3 | 0,3 |
| 66 | 6,2 | 0,0 | 2,6 | 0,6 | 87,3 | 0,0 | 0,7 | 2,3 | 0,3 |
| 73 | 6,5 | 0,0 | 2,9 | 0,7 | 85,9 | 0,0 | 0,9 | 2,8 | 0,2 |
| 90 | 6,5 | 0,0 | 2,8 | 0,6 | 86,6 | 0,0 | 0,5 | 2,8 | 0,1 |
| 97 | 6,5 | 0,0 | 3,0 | 0,7 | 86,0 | 0,0 | 0,8 | 2,9 | 0,2 |
| 162 | 6,3 | 0,0 | 3,5 | 0,7 | 85,3 | 0,0 | 0,6 | 3,5 | 0,1 |
| 169 | 6,5 | 0,0 | 3,3 | 0,7 | 85,6 | 0,0 | 0,6 | 3,2 | 0,1 |
| 186 | 6,6 | 0,0 | 3,3 | 0,7 | 85,7 | 0,0 | 0,5 | 3,2 | 0,1 |
| 193 | 6,5 | 0,0 | 3,3 | 0,7 | 85,7 | 0,0 | 0,5 | 3,3 | 0,0 |
| 210 | 6,6 | 0,0 | 3,4 | 0,7 | 85,7 | 0,0 | 0,5 | 3,0 | 0,0 |
| 217 | 6,3 | 0,0 | 3,5 | 0,7 | 85,4 | 0,0 | 0,5 | 3,6 | 0,0 |
| 234 | 5,8 | 0,0 | 3,4 | 0,5 | 87,3 | 0,0 | 0,4 | 2,6 | 0,0 |
| 241 | 5,8 | 0,0 | 3,4 | 0,5 | 87,1 | 0,0 | 0,6 | 2,3 | 0,2 |
| 260 | 5,7 | 0,0 | 3,4 | 0,4 | 87,3 | 0,0 | 0,6 | 2,3 | 0,2 |
| 330 | 5,8 | 0,0 | 4,2 | 0,5 | 85,9 | 0,0 | 0,6 | 2,3 | 0,7 |
| 337 | 5,8 | 0,0 | 4,1 | 0,4 | 86,4 | 0,0 | 0,6 | 2,6 | 0,0 |
| 354 | 5,9 | 0,0 | 4,2 | 0,4 | 86,4 | 0,0 | 0,5 | 2,5 | 0,0 |
| 361 | 5,8 | 0,0 | 4,4 | 0,5 | 86,0 | 0,0 | 0,7 | 2,5 | 0,2 |
| 378 | 5,9 | 0,0 | 4,4 | 0,3 | 86,3 | 0,0 | 0,5 | 2,6 | 0,0 |
| 385 | 6,0 | 0,0 | 4,4 | 0,3 | 86,2 | 0,0 | 0,5 | 2,6 | 0,0 |
| 402 | 6,0 | 0,0 | 4,5 | 0,3 | 85,7 | 0,0 | 0,5 | 2,8 | 0,0 |
| 409 | 6,0 | 0,0 | 4,7 | 0,4 | 86,5 | 0,0 | 0,2 | 2,1 | 0,0 |
| 426 | 5,9 | 0,0 | 4,7 | 0,4 | 86,1 | 0,0 | 0,6 | 2,5 | 0,0 |
| 433 | 5,9 | 0,0 | 5,0 | 0,4 | 86,5 | 0,0 | 0,0 | 2,2 | 0,0 |
| 498 | 5,9 | 0,0 | 5,1 | 0,3 | 85,6 | 0,0 | 0,5 | 2,6 | 0,0 |
| 505 | 5,9 | 0,0 | 5,3 | 0,3 | 85,3 | 0,0 | 0,6 | 2,7 | 0,0 |
| 522 | 5,8 | 0,0 | 5,6 | 0,5 | 84,8 | 0,0 | 0,6 | 2,6 | 0,0 |
| 529 | 5,9 | 0,0 | 5,3 | 0,4 | 85,2 | 0,0 | 0,6 | 2,7 | 0,0 |
| 546 | 5,7 | 0,0 | 5,6 | 0,6 | 84,8 | 0,0 | 0,6 | 2,6 | 0,0 |
| 553 | 5,7 | 0,0 | 5,8 | 0,6 | 84,5 | 0,0 | 0,5 | 2,8 | 0,0 |
| 570 | 5,5 | 0,0 | 5,4 | 0,3 | 85,8 | 0,0 | 0,4 | 2,5 | 0,0 |
| 577 | 5,7 | 0,0 | 5,6 | 0,3 | 85,6 | 0,0 | 0,3 | 2,4 | 0,0 |
| 593 | 5,7 | 0,0 | 5,9 | 0,4 | 85,1 | 0,0 | 0,4 | 2,4 | 0,0 |
| 601 | 5,7 | 0,0 | 5,7 | 0,3 | 85,4 | 0,0 | 0,4 | 2,5 | 0,1 |
| 673 | 5,6 | 0,0 | 5,9 | 0,3 | 85,5 | 0,0 | 0,6 | 2,1 | 0,1 |
| 690 | 5,7 | 0,0 | 6,0 | 0,3 | 84,6 | 0,0 | 0,6 | 2,8 | 0,0 |
| 714 | 5,7 | 0,0 | 4,5 | 0,3 | 86,3 | 0,0 | 0,6 | 2,4 | 0,1 |
| 721 | 5,6 | 0,0 | 4,4 | 0,2 | 86,4 | 0,0 | 0,6 | 2,7 | 0,0 |
| 738 | 5,5 | 0,0 | 4,4 | 0,2 | 86,5 | 0,0 | 0,7 | 2,7 | 0,0 |
| 837 | 5,7 | 0,0 | 4,9 | 0,2 | 86,1 | 0,0 | 0,3 | 2,4 | 0,4 |
| 845 | 5,7 | 0,0 | 5,0 | 0,3 | 85,9 | 0,0 | 0,4 | 2,4 | 0,4 |
| 862 | 5,5 | 0,0 | 5,0 | 0,2 | 86,1 | 0,0 | 0,3 | 2,4 | 0,4 |
| 869 | 5,5 | 0,0 | 5,2 | 0,3 | 85,8 | 0,0 | 0,0 | 2,8 | 0,3 |
| 886 | 5,6 | 0,0 | 5,2 | 0,3 | 86,1 | 0,0 | 0,1 | 2,3 | 0,4 |
| 893 | 5,6 | 0,0 | 5,4 | 0,2 | 85,9 | 0,0 | 0,2 | 2,4 | 0,4 |
| 908 | 5,6 | 0,0 | 5,4 | 0,2 | 85,9 | 0,0 | 0,2 | 2,4 | 0,4 |
| 916 | 5,6 | 0,0 | 5,6 | 0,3 | 84,9 | 0,0 | 0,4 | 2,7 | 0,3 |
| 933 | 5,7 | 0,0 | 5,6 | 0,3 | 85,0 | 0,0 | 0,4 | 2,7 | 0,3 |
| 941 | 5,4 | 0,0 | 5,6 | 0,2 | 85,8 | 0,0 | 0,2 | 2,4 | 0,3 |
| 1006 | 5,6 | 0,0 | 6,3 | 0,0 | 85,1 | 0,0 | 0,3 | 2,3 | 0,3 |
| 1013 | 5,7 | 0,0 | 6,3 | 0,0 | 85,2 | 0,0 | 0,3 | 2,2 | 0,3 |
| 1030 | 5,4 | 0,0 | 6,1 | 0,0 | 86,6 | 0,0 | 0,0 | 1,8 | 0,0 |
| 1037 | 5,7 | 0,0 | 8,9 | 0,0 | 81,8 | 0,0 | 0,5 | 3,1 | 0,0 |
| 1054 | 5,8 | 0,0 | 9,0 | 0,0 | 81,6 | 0,0 | 0,5 | 3,1 | 0,0 |
| 1063 | 5,5 | 0,0 | 5,8 | 0,0 | 85,0 | 0,0 | 0,5 | 3,2 | 0,0 |
| 1077 | 5,5 | 0,0 | 5,8 | 0,0 | 85,0 | 0,0 | 0,5 | 3,1 | 0,0 |
| 1085 | 5,6 | 0,0 | 5,9 | 0,0 | 84,9 | 0,0 | 0,5 | 3,1 | 0,0 |
| 1100 | 5,6 | 0,0 | 6,0 | 0,0 | 84,2 | 0,0 | 0,5 | 3,6 | 0,0 |
| 1109 | 5,7 | 0,0 | 6,1 | 0,0 | 84,0 | 0,0 | 0,4 | 3,7 | 0,1 |
| 1170 | 5,7 | 0,0 | 6,2 | 0,0 | 84,0 | 0,0 | 0,4 | 3,7 | 0,1 |

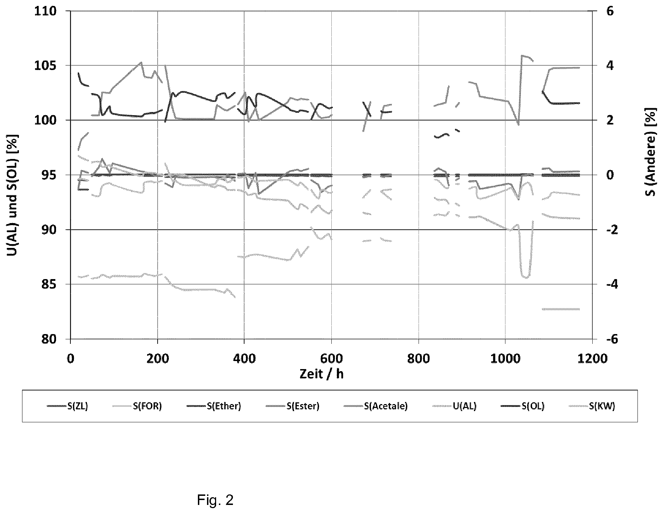

Figur 2 zeigt ein entsprechendes Umsatz-Selektivitäts-Diagramm

### Beispiel 3

Ein Reaktionsaustrag aus der Rhodium-katalysierten Hydroformylierung von Dibuten wurde in einer Kreislaufapparatur bei 180°C und 25 bar absolut an 150 g Katalysator in der Flüssigphase kontinuierlich hydriert. Es wurden stündlich 0,1 l Edukt bei einem Kreislauf von 20 l/h durchgesetzt. Die Abgasmenge betrug 0,5 NL/h. Es wurde ein nach Beispiel 0 hergestellter, chromfreier Ni/Cu-Katalysator auf Al₂O₃-Trägermaterial verwendet. Die Zusammensetzung des verwendeten Einsatzgemisches ist in Tabelle 4 dargestellt.

**Tabelle 4: Zusammensetzung des Einsatzgemisches enthaltend C9-Aldehyde**

| Komponente | KW^{[a]} | ZL^{[b]} | AL^{[c]} | FOR^{[d]} | OL^{[e]} | Ether^{[f]} | Ester^{[g]} | Acetale^{[h]} | Wasser | Rest |
|---|---|---|---|---|---|---|---|---|---|---|
| Gehalt [Ma.%] | 6,5 | 0,1 | 90,3 | 0,6 | 2,5 | 0,0 | 0,0 | 0,1 | 0,0 | 0,0 |

| | | | | | | | | | | |
|---|---|---|---|---|---|---|---|---|---|---|
| [a] KW: Kohlenwasserstoffe; [b]: Zwischenlauf; [c]: C₉-Aldehyde; [d]: C₉-Formiate; [e]: C9-Alkohole; [f]: C₉/C₉-Ether; [g]: C₉/C₉-Ester; [h]: C₉/C₉/C₉-Acetale | | | | | | | | | | |

Der zeitliche Verlauf der Zusammensetzung des Produktgemisches ist in Tabelle 5 wiedergegen:

**Tabelle 5: Zeitlicher Verlauf der Zusammensetzung des Produktgemisches**

| t [h] | KW^{[a]} | ZL^{[b]} | AL^{[c]} | FOR^{[d]} | OL^{[e]} | Ether^{[f]} | Ester^{[g]} | Acetale^{[h]} | Wasser | Rest |
|---|---|---|---|---|---|---|---|---|---|---|
| 0 | 6,4 | 0,1 | 59,1 | 0,0 | 32,9 | 0,5 | 0,7 | 0,2 | 0,0 | 0,1 |
| 1 | 6,3 | 0,1 | 0,9 | 0,0 | 91,8 | 0,3 | 0,4 | 0,2 | 0,0 | 0,1 |
| 2 | 6,4 | 0,1 | 0,0 | 0,0 | 92,4 | 0,3 | 0,4 | 0,2 | 0,0 | 0,1 |
| 3 | 6,4 | 0,1 | 0,1 | 0,0 | 92,4 | 0,4 | 0,4 | 0,1 | 0,0 | 0,1 |
| 5 | 6,3 | 0,1 | 0,1 | 0,0 | 92,3 | 0,4 | 0,7 | 0,0 | 0,0 | 0,1 |
| 22 | 6,4 | 0,1 | 0,0 | 0,0 | 92,4 | 0,5 | 0,5 | 0,0 | 0,0 | 0,1 |
| 46 | 6,4 | 0,0 | 0,0 | 0,0 | 92,5 | 0,6 | 0,4 | 0,0 | 0,0 | 0,0 |
| 70 | 6,4 | 0,0 | 0,0 | 0,0 | 91,9 | 1,3 | 0,4 | 0,0 | 0,0 | 0,0 |
| 142 | 6,4 | 0,2 | 0,0 | 0,1 | 91,0 | 1,9 | 0,3 | 0,0 | 0,0 | 0,0 |
| 166 | 6,4 | 0,0 | 0,0 | 0,0 | 91,3 | 2,0 | 0,3 | 0,0 | 0,0 | 0,0 |
| 190 | 6,4 | 0,3 | 0,0 | 0,1 | 90,4 | 2,4 | 0,3 | 0,0 | 0,0 | 0,0 |
| 214 | 6,4 | 0,0 | 0,0 | 0,0 | 91,0 | 2,2 | 0,3 | 0,0 | 0,0 | 0,0 |
| 238 | 6,4 | 0,0 | 0,0 | 0,0 | 90,9 | 2,3 | 0,3 | 0,0 | 0,0 | 0,0 |
| 310 | 6,3 | 0,1 | 0,0 | 0,0 | 90,7 | 2,6 | 0,3 | 0,0 | 0,0 | 0,0 |
| 335 | 6,2 | 0,1 | 0,0 | 0,0 | 90,8 | 2,7 | 0,2 | 0,0 | 0,0 | 0,0 |
| 358 | 6,2 | 0,1 | 0,0 | 0,0 | 90,7 | 2,8 | 0,2 | 0,0 | 0,0 | 0,0 |
| 382 | 6,2 | 0,0 | 0,0 | 0,0 | 90,7 | 2,8 | 0,2 | 0,0 | 0,0 | 0,0 |
| 406 | 6,2 | 0,1 | 0,0 | 0,0 | 90,6 | 2,9 | 0,2 | 0,0 | 0,0 | 0,0 |
| 478 | 6,1 | 0,1 | 0,0 | 0,0 | 90,5 | 3,0 | 0,2 | 0,0 | 0,0 | 0,0 |
| 502 | 6,2 | 0,1 | 0,0 | 0,0 | 90,5 | 3,0 | 0,2 | 0,0 | 0,0 | 0,0 |
| 526 | 6,2 | 0,1 | 0,0 | 0,0 | 90,6 | 3,0 | 0,2 | 0,0 | 0,0 | 0,0 |
| 550 | 6,2 | 0,1 | 0,0 | 0,0 | 90,5 | 3,0 | 0,2 | 0,0 | 0,0 | 0,0 |
| 575 | 6,1 | 0,1 | 0,0 | 0,0 | 90,6 | 3,0 | 0,2 | 0,0 | 0,0 | 0,0 |
| 646 | 6,2 | 0,1 | 0,0 | 0,0 | 90,5 | 3,0 | 0,2 | 0,0 | 0,0 | 0,0 |
| 670 | 6,2 | 0,1 | 0,0 | 0,0 | 90,8 | 2,8 | 0,1 | 0,0 | 0,0 | 0,0 |
| 694 | 6,2 | 0,1 | 0,0 | 0,0 | 90,5 | 3,0 | 0,2 | 0,0 | 0,0 | 0,0 |
| 718 | 6,2 | 0,1 | 0,0 | 0,0 | 90,5 | 3,0 | 0,2 | 0,0 | 0,0 | 0,0 |
| 743 | 6,2 | 0,1 | 0,0 | 0,0 | 90,6 | 3,0 | 0,2 | 0,0 | 0,0 | 0,0 |
| 814 | 6,2 | 0,1 | 0,0 | 0,0 | 90,6 | 2,9 | 0,2 | 0,0 | 0,0 | 0,0 |
| 838 | 6,2 | 0,1 | 0,0 | 0,0 | 90,6 | 2,9 | 0,2 | 0,0 | 0,0 | 0,0 |
| 863 | 6,2 | 0,1 | 0,0 | 0,0 | 90,7 | 2,9 | 0,2 | 0,0 | 0,0 | 0,0 |
| 982 | 6,3 | 0,1 | 0,0 | 0,0 | 90,7 | 2,8 | 0,1 | 0,0 | 0,0 | 0,0 |
| 1009 | 6,4 | 0,1 | 0,0 | 0,0 | 92,3 | 1,3 | 0,0 | 0,0 | 0,0 | 0,0 |
| 1054 | 6,2 | 0,1 | 0,0 | 0,0 | 90,9 | 2,7 | 0,1 | 0,0 | 0,0 | 0,0 |

### Beispiel 4

Reines 1-Nonanal wurde in einer Rieselbettapparatur bei einer Temperatur von 180°C und einem Druck 25*10⁵ Pa absolut an 3 g Katalysator in der Flüssigphase kontinuierlich hydriert. Es wurden stündlich 0,12 l Edukt durchgesetzt. Die Abgasmenge betrug 20 NL/h. Es wurde ein nach Beispiel 0 hergestellter, chromfreier Ni/Cu-Katalysator auf Al₂O₃-Trägermaterial verwendet. Allerdings wurde der Katalysator als Pulver eingesetzt, um Stofftransportproblemen zu begegnen. Skalierbarkeit auf technische Pellets war aber weiterhin gegeben. Der zeitliche Verlauf der Zusammensetzung des Produktgemisches ist in Tabelle 6 wiedergegen:

**Tabelle 6: Zeitlicher Verlauf der Zusammensetzung des Produktgemisches gemäß Beispiel 4**

| t [h] | KW | AL | FOR | OL | Ether | Säure | Einfach hydriertes Enal | Ester | Enal | Hydriertes Enal | Acetale | HS |
|---|---|---|---|---|---|---|---|---|---|---|---|---|
| 18 | 0,21 | 0,09 | 0,05 | 85,73 | 0,49 | 0,12 | 0,01 | 2,29 | 0,27 | 4,86 | 0,06 | 5,08 |
| 42 | 0,18 | 0,05 | 0,04 | 86,75 | 0,27 | 0,10 | 0,01 | 2,42 | 0,19 | 4,59 | 0,07 | 4,60 |
| 66 | 0,16 | 0,04 | 0,04 | 85,39 | 0,36 | 0,11 | 0,00 | 2,83 | 0,18 | 5,19 | 0,09 | 4,68 |
| 144 | 0,26 | 0,03 | 0,04 | 83,86 | 0,89 | 0,08 | 0,00 | 3,13 | 0,36 | 4,97 | 0,16 | 4,97 |
| 162 | 0,24 | 0,10 | 0,04 | 84,55 | 0,85 | 0,05 | 0,00 | 3,05 | 0,30 | 4,69 | 0,15 | 4,91 |
| 186 | 0,20 | 0,05 | 0,04 | 83,42 | 0,78 | 0,03 | 0,00 | 3,56 | 0,27 | 5,23 | 0,13 | 4,98 |
| 210 | 0,20 | 0,05 | 0,04 | 82,93 | 0,78 | 0,03 | 0,00 | 3,88 | 0,29 | 5,49 | 0,15 | 4,70 |
| 234 | 0,19 | 0,04 | 0,04 | 82,52 | 0,85 | 0,03 | 0,00 | 3,94 | 0,27 | 5,44 | 0,17 | 5,14 |
| 306 | 0,19 | 0,04 | 0,04 | 82,85 | 0,78 | 0,02 | 0,00 | 3,93 | 0,24 | 5,32 | 0,17 | 5,07 |
| 330 | 0,18 | 0,04 | 0,00 | 83,41 | 0,80 | 0,03 | 0,00 | 4,02 | 0,23 | 5,40 | 0,18 | 4,29 |
| 354 | 0,17 | 0,04 | 0,00 | 82,53 | 0,81 | 0,02 | 0,00 | 4,16 | 0,21 | 5,56 | 0,18 | 4,96 |

| | | | | | | | | | | | | |
|---|---|---|---|---|---|---|---|---|---|---|---|---|
| t: Zeit, KW: sonstige Kohlenwasserstoffe, AL: Aldehyde, FOR: Formiate, OL: Alkohol, HS: Hochsieder | | | | | | | | | | | | |

### Beispiel 5 (nicht erfindungsgemäß)

Der in Beispiel 4 beschriebene Versuch wurde analog mit demselben Edukt und unter denselben Bedingungen durchgeführt. Es wurde jedoch ein chromhaltiger Katalysator eingesetzt, wie er auch für die in DE19842370A1 beschrieben Versuche verwendet wurde. Der zeitliche Verlauf der Zusammensetzung des Produktgemisches ist in Tabelle 7 wiedergegeben:

**Tabelle 7: Zeitlicher Verlauf der Zusammensetzung des Produktgemisches gemäß Beispiel 5**

| t [h] | KW | AL | FOR | OL | Ether | Säure | Einfach hydriertes Enal | Ester | Enal | Hydriertes Enal | Acetale | HS |
|---|---|---|---|---|---|---|---|---|---|---|---|---|
| 18,0 | 0,16 | 0,39 | 0,04 | 83,39 | 0,41 | 0,03 | 0,00 | 2,49 | 0,36 | 4,89 | 0,08 | 6,86 |
| 42,0 | 0,16 | 0,07 | 0,03 | 84,82 | 0,39 | 0,02 | 0,01 | 2,41 | 0,23 | 4,09 | 0,26 | 6,71 |
| 66,0 | 0,15 | 0,05 | 0,03 | 84,77 | 0,45 | 0,02 | 0,01 | 2,52 | 0,20 | 4,53 | 0,14 | 6,16 |
| 138,0 | 0,29 | 0,71 | 0,04 | 83,45 | 0,70 | 0,16 | 0,00 | 2,62 | 0,51 | 4,05 | 0,19 | 6,22 |
| 144,0 | 0,20 | 0,02 | 0,03 | 84,28 | 0,77 | 0,05 | 0,00 | 2,64 | 0,28 | 4,10 | 0,11 | 6,57 |
| 162,0 | 0,21 | 0,05 | 0,03 | 84,85 | 0,72 | 0,02 | 0,00 | 2,60 | 0,27 | 4,07 | 0,12 | 6,07 |
| 186,0 | 0,18 | 0,05 | 0,03 | 83,48 | 0,69 | 0,03 | 0,00 | 3,09 | 0,25 | 4,67 | 0,16 | 5,98 |
| 210,0 | 0,17 | 0,04 | 0,03 | 83,14 | 0,69 | 0,02 | 0,00 | 3,37 | 0,23 | 4,79 | 0,17 | 5,88 |
| 234,0 | 0,17 | 0,04 | 0,04 | 84,37 | 0,68 | 0,02 | 0,00 | 3,17 | 0,21 | 4,37 | 0,15 | 5,43 |
| 306,0 | 0,19 | 0,04 | 0,00 | 83,27 | 0,71 | 0,02 | 0,00 | 3,54 | 0,07 | 4,71 | 0,18 | 5,76 |
| 330,0 | 0,18 | 0,03 | 0,00 | 82,68 | 0,73 | 0,02 | 0,00 | 3,81 | 0,08 | 5,06 | 0,19 | 5,69 |
| 354,0 | 0,18 | 0,04 | 0,00 | 83,00 | 0,72 | 0,02 | 0,00 | 3,67 | 0,07 | 4,91 | 0,19 | 5,64 |

| | | | | | | | | | | | | |
|---|---|---|---|---|---|---|---|---|---|---|---|---|
| t: Zeit, KW: sonstige Kohlenwasserstoffe, AL: Aldehyde, FOR: Formiate, OL: Alkohol, HS: Hochsieder | | | | | | | | | | | | |

### Beispiel 6

Ein Reaktionsaustrag aus der Cobalt-katalysierten Hydroformylierung von Buten wurde in einer Rieselbettapparatur bei 120°C und 25 bar absolut an 3 g Katalysator in der Flüssigphase kontinuierlich hydriert. Es wurden stündlich 0,12 l Edukt eingesetzt. Die Abgasmenge betrug 20 NL/h. Es wurde ein nach Beispiel 0 hergestellter, chromfreier Ni/Cu-Katalysator auf Al2O3-Trägermaterial verwendet. Allerdings wurde der Katalysator als Pulver eingesetzt, um Stofftransportproblemen zu begegnen. Skalierbarkeit auf technische Pellets war aber weiterhin gegeben. Der zeitliche Verlauf der Zusammensetzung des Produktgemisches ist in Tabelle 8 wiedergegen; die Zusammensetzung des Eduktgemisches entspricht dabei dem Tabelleneintrag zum Zeitpunkt t=0.

**Tabelle 8: Zeitlicher Verlauf der Zusammensetzung des Produktgemisches gemäß Beispiel 6**

| t[h] | Alkane | 2-Methyl-1-Butanal | n-Pentanal | 2-Methyl-1-butanol | n-Pentanol | Pentansäure | 2-Propyl-2-heptenal | Unbekannte |
|---|---|---|---|---|---|---|---|---|
| 0 | 0,57 | 4,59 | 86,66 | 0,00 | 0,12 | 0,12 | 1,29 | 4,57 |
| 16,4 | 0,08 | 0,10 | 0,29 | 4,32 | 75,71 | 0,15 | 5,40 | 11,60 |
| 88,7 | 0,09 | 0,01 | 0,01 | 4,71 | 83,60 | 0,00 | 0,41 | 10,08 |
| 114,1 | 0,09 | 0,05 | 0,15 | 4,61 | 82,57 | 0,15 | 0,74 | 10,24 |
| 138,3 | 0,09 | 0,14 | 1,74 | 4,18 | 76,17 | 0,20 | 0,77 | 13,29 |
| 184,8 | 0,09 | 0,33 | 2,57 | 4,03 | 75,13 | 0,22 | 0,70 | 14,74 |
| 256,6 | 0,09 | 0,14 | 0,59 | 4,51 | 82,42 | 0,24 | 0,15 | 10,10 |
| 286,7 | 0,09 | 0,16 | 0,76 | 4,41 | 82,12 | 0,26 | 0,07 | 10,90 |

### Beispiel 7 (nicht erfindungsgemäß)

Der in Beispiel 6 beschriebene Versuch wurde analog mit demselben Edukt und unter denselben Bedingungen durchgeführt. Es wurde jedoch ein chromhaltiger Katalysator eingesetzt, wie er auch für die in DE19842370A1 beschrieben Versuche verwendet wurde. Der zeitliche Verlauf der Zusammensetzung des Produktgemisches ist in Tabelle 9 wiedergegen; die Zusammensetzung des Eduktgemisches entspricht dabei dem Tabelleneintrag zum Zeitpunkt t=0.

**Tabelle 9: Zeitlicher Verlauf der Zusammensetzung des Produktgemisches gemäß Beispiel 7**

| t[h] | Alkane | 2-Methyl-1-Butanal | n-Pentanal | 2-Methyl-1-butanol | n-Pentanol | Pentansäure | 2-Propyl-2-heptenal | Unbekannte |
|---|---|---|---|---|---|---|---|---|
| 0 | 0,57 | 4,59 | 86,66 | 0,00 | 0,12 | 0,12 | 1,29 | 4,57 |
| 16,4 | 0,02 | 0,06 | 0,39 | 1,09 | 19,23 | 0,00 | 1,02 | 3,14 |
| 88,7 | 0,11 | 0,18 | 0,90 | 4,46 | 79,31 | 0,19 | 0,56 | 12,34 |
| 114,1 | 0,11 | 0,12 | 0,26 | 4,52 | 79,57 | 0,21 | 0,63 | 12,73 |
| 138,3 | 0,12 | 0,30 | 1,33 | 4,26 | 78,78 | 0,21 | 0,57 | 11,57 |
| 184,8 | 0,11 | 0,56 | 2,45 | 3,94 | 77,56 | 0,27 | 0,64 | 11,68 |
| 256,6 | 0,13 | 1,09 | 4,18 | 3,48 | 77,77 | 0,26 | 1,18 | 7,28 |
| 286,7 | 0,13 | 1,28 | 5,80 | 3,31 | 76,24 | 0,27 | 1,06 | 9,45 |

### Beispiel 8

Ein Gemisch aus Valeraldehyd und 2-Propylheptanal wurde einer kontinuierlichen Hydrierung an einem gemäß Beispiel 0 hergestellten Katalysator unterworfen. Die Katalysatormenge betrug 60,9 g. Die Temperatur betrug 180°C, der Druck 25*10⁵ Pa. Die Eduktzufuhr betrug 0.12 l/h. Der Kreislauf wurde auf 45 l/h eingestellt. Die abgeführte Abgasmenge betrug 1 Nl/min. Der zeitliche Verlauf der Zusammensetzung des Produktgemisches ist in Tabelle 10 wiedergegen; die Zusammensetzung des Eduktgemisches entspricht dabei dem Tabelleneintrag zum Zeitpunkt t=0.

**Tabelle 10: Zeitlicher Verlauf der Zusammensetzung des Produktgemisches gemäß Beispiel 8**

| t[h] | 2-Methylbutanal | n-Pentanal | 2-Me-1-Butanol | n-Pentanol | 4-Me-2-Pr-2-Hexenal | 4-Me-2-Pr-2-Hexanal | 4-Me-2-Pr-Hexenol | 4-Me-2-Pr-Hexanol | 2-Pr-2-Heptenal (1) uvz | 2-Pr-2-Heptenal (2) vz | 2-PH-ol | 2-PHE-ol | Unbekannte |
|---|---|---|---|---|---|---|---|---|---|---|---|---|---|
| 0 | 1,64 | 13,18 | 0,01 | 0,14 | 1,91 | 0,95 | 0,00 | 0,00 | 0,00 | 81,50 | 0,00 | 0,00 | 0,67 |
| 0 | 8,87 | 70,84 | 0,04 | 0,78 | 10,37 | 5,22 | 0,06 | 0,00 | 0,00 | 0,27 | 0,00 | 0,00 | 3,55 |
| 0 | 0,01 | 0,07 | 1,66 | 12,64 | 0,01 | 0,40 | 1,73 | 0,26 | 0,47 | 0,04 | 81,91 | 0,00 | 0,80 |
| 17 | 0,01 | 0,11 | 1,69 | 13,07 | 0,01 | 0,33 | 1,73 | 0,13 | 0,66 | 0,06 | 81,49 | 0,00 | 0,72 |
| 89 | 0,01 | 0,06 | 1,71 | 13,26 | 0,02 | 0,42 | 1,73 | 0,07 | 0,32 | 0,02 | 81,75 | 0,00 | 0,62 |
| 96 | 0,01 | 0,06 | 1,71 | 13,20 | 0,02 | 0,44 | 1,73 | 0,07 | 0,33 | 0,03 | 81,77 | 0,00 | 0,62 |
| 113 | 0,01 | 0,06 | 1,71 | 13,19 | 0,02 | 0,43 | 1,73 | 0,07 | 0,32 | 0,02 | 81,81 | 0,00 | 0,62 |
| 120 | 0,01 | 0,06 | 1,71 | 13,19 | 0,02 | 0,43 | 1,73 | 0,07 | 0,32 | 0,02 | 81,80 | 0,00 | 0,62 |
| 137 | 0,01 | 0,06 | 1,72 | 13,22 | 0,02 | 0,44 | 1,73 | 0,08 | 0,32 | 0,02 | 81,75 | 0,00 | 0,63 |
| 144 | 0,01 | 0,06 | 1,72 | 13,21 | 0,02 | 0,44 | 1,73 | 0,07 | 0,32 | 0,02 | 81,76 | 0,00 | 0,63 |
| 161 | 0,01 | 0,06 | 1,71 | 13,20 | 0,02 | 0,45 | 1,73 | 0,07 | 0,32 | 0,03 | 81,77 | 0,00 | 0,63 |
| 167 | 0,01 | 0,06 | 1,72 | 13,29 | 0,02 | 0,46 | 1,73 | 0,07 | 0,31 | 0,02 | 81,67 | 0,00 | 0,63 |
| 188 | 0,01 | 0,06 | 1,72 | 13,28 | 0,03 | 0,45 | 1,73 | 0,07 | 0,32 | 0,02 | 81,68 | 0,00 | 0,62 |
| 192 | 0,01 | 0,06 | 1,71 | 13,25 | 0,03 | 0,46 | 1,73 | 0,07 | 0,33 | 0,03 | 81,70 | 0,00 | 0,62 |
| 257 | 0,01 | 0,07 | 1,71 | 13,27 | 0,03 | 0,48 | 1,73 | 0,07 | 0,34 | 0,03 | 81,66 | 0,00 | 0,61 |
| 281 | 0,01 | 0,05 | 1,69 | 10,87 | 0,03 | 0,50 | 1,78 | 0,06 | 0,36 | 0,03 | 84,01 | 0,00 | 0,61 |
| 288 | 0,01 | 0,05 | 1,69 | 10,59 | 0,03 | 0,39 | 1,79 | 0,06 | 0,35 | 0,03 | 84,42 | 0,00 | 0,61 |
| 306 | 0,01 | 0,05 | 1,62 | 13,38 | 0,03 | 0,48 | 0,07 | 1,73 | 0,06 | 0,35 | 81,65 | 0,00 | 0,59 |
| 312 | 0,01 | 0,05 | 1,68 | 10,34 | 0,03 | 0,51 | 1,79 | 0,06 | 0,36 | 0,03 | 84,54 | 0,00 | 0,61 |
| 329 | 0,01 | 0,05 | 1,68 | 10,36 | 0,01 | 0,51 | 1,79 | 0,05 | 0,37 | 0,03 | 84,54 | 0,00 | 0,59 |
| 335 | 0,01 | 0,06 | 1,68 | 10,40 | 0,01 | 0,51 | 1,79 | 0,05 | 0,37 | 0,03 | 84,50 | 0,00 | 0,59 |
| 359 | 0,01 | 0,07 | 1,71 | 13,33 | 0,01 | 0,49 | 1,73 | 0,06 | 0,35 | 0,03 | 81,61 | 0,00 | 0,60 |
| 425 | 0,01 | 0,08 | 1,73 | 14,45 | 0,01 | 0,50 | 1,70 | 0,06 | 0,37 | 0,03 | 80,49 | 0,00 | 0,57 |
| 432 | 0,01 | 0,07 | 1,73 | 14,42 | 0,01 | 0,51 | 1,71 | 0,06 | 0,37 | 0,03 | 80,50 | 0,00 | 0,57 |
| 473 | 0,01 | 0,07 | 1,72 | 13,25 | 0,01 | 0,53 | 1,73 | 0,06 | 0,38 | 0,03 | 81,62 | 0,00 | 0,59 |
| 480 | 0,01 | 0,07 | 1,72 | 13,25 | 0,01 | 0,52 | 1,73 | 0,06 | 0,38 | 0,03 | 81,64 | 0,00 | 0,58 |
| 497 | 0,01 | 0,07 | 1,72 | 13,25 | 0,01 | 0,53 | 1,73 | 0,06 | 0,38 | 0,03 | 81,65 | 0,00 | 0,57 |
| 504 | 0,01 | 0,07 | 1,72 | 13,34 | 0,01 | 0,52 | 1,73 | 0,06 | 0,37 | 0,03 | 81,57 | 0,00 | 0,57 |
| 593 | 0,01 | 0,08 | 1,71 | 13,46 | 0,01 | 0,54 | 1,72 | 0,06 | 0,40 | 0,03 | 81,41 | 0,00 | 0,57 |
| 601 | 0,01 | 0,08 | 1,71 | 13,46 | 0,01 | 0,54 | 1,72 | 0,06 | 0,40 | 0,03 | 81,42 | 0,00 | 0,56 |
| 617 | 0,01 | 0,08 | 1,71 | 13,46 | 0,01 | 0,55 | 0,06 | 1,73 | 0,39 | 0,03 | 81,55 | 0,00 | 0,42 |
| 637 | 0,02 | 0,05 | 3,11 | 9,38 | 0,04 | 0,53 | 3,48 | 0,05 | 0,40 | 0,03 | 82,34 | 0,00 | 0,58 |
| 644 | 0,02 | 0,05 | 3,25 | 9,00 | 0,04 | 0,53 | 3,65 | 0,05 | 0,40 | 0,03 | 82,40 | 0,00 | 0,58 |
| 661 | 0,02 | 0,05 | 3,35 | 8,68 | 0,04 | 0,54 | 3,79 | 0,04 | 0,40 | 0,03 | 82,46 | 0,00 | 0,59 |
| 684 | 0,02 | 0,08 | 3,20 | 12,43 | 0,03 | 0,55 | 3,47 | 0,05 | 0,40 | 0,03 | 79,18 | 0,00 | 0,57 |
| 692 | 0,02 | 0,08 | 3,18 | 12,93 | 0,03 | 0,53 | 3,43 | 0,06 | 0,40 | 0,03 | 78,74 | 0,00 | 0,58 |
| 780 | 0,02 | 0,03 | 3,36 | 6,86 | 0,04 | 0,57 | 3,87 | 0,05 | 0,44 | 0,03 | 84,12 | 0,00 | 0,60 |
| 787 | 0,02 | 0,03 | 3,39 | 6,03 | 0,04 | 0,58 | 3,94 | 0,04 | 0,44 | 0,03 | 84,85 | 0,00 | 0,61 |
| 804 | 0,02 | 0,03 | 3,42 | 5,36 | 0,04 | 0,59 | 3,99 | 0,03 | 0,44 | 0,03 | 85,42 | 0,00 | 0,63 |
| 807 | 0,02 | 0,03 | 3,42 | 5,27 | 0,04 | 0,58 | 3,99 | 0,03 | 0,43 | 0,03 | 85,51 | 0,00 | 0,63 |
| 824 | 0,02 | 0,07 | 3,34 | 7,97 | 0,04 | 0,56 | 3,80 | 0,04 | 0,40 | 0,03 | 83,10 | 0,00 | 0,63 |
| 832 | 0,02 | 0,09 | 3,30 | 11,29 | 0,04 | 0,54 | 3,63 | 0,05 | 0,40 | 0,03 | 80,00 | 0,00 | 0,62 |
| 848 | 0,02 | 0,09 | 3,30 | 14,31 | 0,04 | 0,54 | 3,51 | 0,06 | 0,40 | 0,03 | 77,12 | 0,00 | 0,58 |
| 855 | 0,02 | 0,09 | 3,30 | 14,71 | 0,03 | 0,53 | 3,50 | 0,06 | 0,40 | 0,03 | 76,76 | 0,00 | 0,57 |
| 944 | 0,02 | 0,09 | 3,29 | 15,15 | 0,04 | 0,55 | 3,48 | 0,06 | 0,41 | 0,03 | 76,32 | 0,00 | 0,55 |
| 968 | 0,02 | 0,08 | 3,62 | 13,57 | 0,04 | 0,57 | 3,59 | 0,06 | 0,42 | 0,03 | 77,45 | 0,00 | 0,54 |
| 976 | 0,02 | 0,08 | 3,65 | 13,27 | 0,04 | 0,57 | 3,61 | 0,06 | 0,43 | 0,03 | 77,70 | 0,00 | 0,54 |
| 992 | 0,02 | 0,08 | 3,70 | 13,19 | 0,04 | 0,58 | 3,62 | 0,06 | 0,43 | 0,03 | 77,72 | 0,00 | 0,54 |
| 1088 | 0,03 | 0,08 | 3,72 | 13,05 | 0,04 | 0,60 | 3,62 | 0,06 | 0,45 | 0,03 | 77,77 | 0,00 | 0,53 |
| 1096 | 0,03 | 0,08 | 3,73 | 13,05 | 0,04 | 0,61 | 3,63 | 0,06 | 0,44 | 0,03 | 77,77 | 0,00 | 0,53 |
| 1112 | 0,03 | 0,08 | 3,72 | 13,04 | 0,04 | 0,61 | 3,63 | 0,06 | 0,45 | 0,03 | 77,77 | 0,00 | 0,53 |
| 1120 | 0,03 | 0,08 | 3,74 | 13,00 | 0,04 | 0,61 | 3,63 | 0,06 | 0,46 | 0,04 | 77,80 | 0,00 | 0,53 |
| 1136 | 0,03 | 0,08 | 3,77 | 12,98 | 0,04 | 0,60 | 3,64 | 0,05 | 0,45 | 0,03 | 77,80 | 0,00 | 0,53 |
| 1143 | 0,03 | 0,09 | 3,77 | 12,99 | 0,04 | 0,61 | 3,64 | 0,05 | 0,47 | 0,04 | 77,75 | 0,00 | 0,52 |
| 1160 | 0,03 | 0,08 | 3,76 | 13,01 | 0,04 | 0,60 | 3,64 | 0,05 | 0,45 | 0,03 | 77,79 | 0,00 | 0,52 |
| 1184 | 0,03 | 0,09 | 3,77 | 12,99 | 0,04 | 0,61 | 3,64 | 0,05 | 0,46 | 0,03 | 77,77 | 0,00 | 0,53 |
| 1191 | 0,03 | 0,08 | 3,77 | 12,98 | 0,04 | 0,60 | 3,64 | 0,05 | 0,45 | 0,03 | 77,80 | 0,00 | 0,53 |
| 1256 | 0,03 | 0,09 | 3,77 | 12,97 | 0,04 | 0,61 | 3,63 | 0,05 | 0,47 | 0,03 | 77,79 | 0,00 | 0,53 |
| 1264 | 0,03 | 0,09 | 3,77 | 12,97 | 0,04 | 0,61 | 3,64 | 0,05 | 0,47 | 0,04 | 77,77 | 0,00 | 0,53 |
| 1280 | 0,03 | 0,09 | 3,76 | 12,98 | 0,04 | 0,62 | 3,64 | 0,05 | 0,47 | 0,04 | 77,77 | 0,00 | 0,53 |
| 1287 | 0,03 | 0,08 | 3,76 | 12,82 | 0,04 | 0,62 | 3,65 | 0,05 | 0,47 | 0,04 | 77,92 | 0,00 | 0,53 |
| 1304 | 0,03 | 0,08 | 3,75 | 12,64 | 0,04 | 0,61 | 3,65 | 0,05 | 0,46 | 0,04 | 78,13 | 0,00 | 0,52 |
| 1311 | 0,03 | 0,08 | 3,75 | 12,63 | 0,04 | 0,61 | 3,66 | 0,05 | 0,47 | 0,04 | 78,11 | 0,00 | 0,53 |
| 1328 | 0,03 | 0,09 | 3,75 | 12,62 | 0,05 | 0,62 | 3,65 | 0,05 | 0,48 | 0,04 | 78,07 | 0,00 | 0,56 |
| 1335 | 0,03 | 0,08 | 3,75 | 12,62 | 0,04 | 0,62 | 3,66 | 0,05 | 0,47 | 0,04 | 78,12 | 0,00 | 0,52 |
| 1352 | 0,03 | 0,09 | 3,75 | 12,63 | 0,04 | 0,62 | 3,66 | 0,05 | 0,49 | 0,04 | 78,08 | 0,00 | 0,53 |
| 1424 | 0,03 | 0,09 | 3,60 | 13,01 | 0,04 | 0,64 | 3,12 | 0,06 | 0,49 | 0,04 | 78,28 | 0,00 | 0,60 |
| 1432 | 0,03 | 0,09 | 3,60 | 13,01 | 0,04 | 0,64 | 3,12 | 0,06 | 0,49 | 0,04 | 78,30 | 0,00 | 0,59 |
| 1448 | 0,03 | 0,09 | 3,60 | 13,02 | 0,04 | 0,64 | 3,12 | 0,06 | 0,49 | 0,04 | 78,29 | 0,00 | 0,60 |
| 1455 | 0,03 | 0,09 | 3,59 | 12,99 | 0,04 | 0,64 | 3,12 | 0,06 | 0,48 | 0,04 | 78,34 | 0,00 | 0,60 |
| 1472 | 0,03 | 0,09 | 3,58 | 12,98 | 0,04 | 0,64 | 3,12 | 0,06 | 0,50 | 0,04 | 78,33 | 0,00 | 0,60 |
| 1502 | 0,03 | 0,09 | 3,58 | 13,02 | 0,04 | 0,63 | 3,05 | 0,09 | 0,49 | 0,04 | 78,22 | 0,00 | 0,72 |
| 1519 | 0,03 | 0,09 | 3,56 | 13,03 | 0,04 | 0,63 | 3,01 | 0,07 | 0,50 | 0,04 | 78,38 | 0,00 | 0,63 |
| 1590 | 0,03 | 0,09 | 3,54 | 13,13 | 0,03 | 0,65 | 2,95 | 0,07 | 0,52 | 0,05 | 78,33 | 0,00 | 0,62 |
| 1597 | 0,03 | 0,09 | 3,53 | 13,10 | 0,03 | 0,65 | 2,95 | 0,06 | 0,51 | 0,04 | 78,38 | 0,00 | 0,62 |
| 1614 | 0,03 | 0,09 | 3,54 | 13,14 | 0,04 | 0,65 | 2,95 | 0,06 | 0,51 | 0,04 | 78,32 | 0,00 | 0,62 |
| 1621 | 0,03 | 0,09 | 3,53 | 13,12 | 0,04 | 0,63 | 2,93 | 0,07 | 0,51 | 0,04 | 78,39 | 0,00 | 0,62 |
| 1638 | 0,03 | 0,09 | 3,53 | 13,20 | 0,04 | 0,65 | 2,91 | 0,07 | 0,51 | 0,04 | 78,31 | 0,00 | 0,63 |
| 1646 | 0,03 | 0,10 | 3,54 | 13,23 | 0,04 | 0,65 | 2,91 | 0,06 | 0,53 | 0,04 | 78,24 | 0,00 | 0,63 |
| 1662 | 0,03 | 0,10 | 3,53 | 13,22 | 0,04 | 0,67 | 2,90 | 0,06 | 0,53 | 0,04 | 78,12 | 0,00 | 0,78 |
| 1691 | 0,03 | 0,09 | 3,52 | 13,09 | 0,04 | 0,65 | 2,90 | 0,07 | 0,53 | 0,05 | 78,26 | 0,00 | 0,78 |
| 1698 | 0,03 | 0,09 | 3,52 | 13,11 | 0,03 | 0,66 | 2,90 | 0,06 | 0,51 | 0,04 | 78,42 | 0,00 | 0,62 |
| 1758 | 0,03 | 0,10 | 3,51 | 13,15 | 0,04 | 0,67 | 2,90 | 0,07 | 0,54 | 0,04 | 78,34 | 0,00 | 0,61 |
| 1781 | 0,03 | 0,10 | 3,53 | 13,15 | 0,04 | 0,68 | 2,90 | 0,06 | 0,55 | 0,04 | 78,31 | 0,00 | 0,61 |
| 1793 | 0,03 | 0,10 | 3,53 | 13,13 | 0,04 | 0,68 | 2,90 | 0,06 | 0,55 | 0,04 | 78,33 | 0,00 | 0,61 |
| 1806 | 0,03 | 0,10 | 3,52 | 13,11 | 0,04 | 0,68 | 2,90 | 0,07 | 0,56 | 0,05 | 78,34 | 0,00 | 0,61 |
| 1813 | 0,03 | 0,10 | 3,53 | 13,13 | 0,04 | 0,67 | 2,90 | 0,06 | 0,55 | 0,05 | 78,33 | 0,00 | 0,62 |
| 1829 | 0,03 | 0,10 | 3,52 | 13,08 | 0,04 | 0,67 | 2,90 | 0,07 | 0,55 | 0,05 | 78,39 | 0,00 | 0,62 |
| 1854 | 0,03 | 0,09 | 3,52 | 13,10 | 0,03 | 0,68 | 2,89 | 0,07 | 0,55 | 0,05 | 78,40 | 0,00 | 0,60 |
| 1865 | 0,03 | 0,10 | 3,52 | 13,09 | 0,03 | 0,67 | 2,89 | 0,07 | 0,56 | 0,04 | 78,38 | 0,00 | 0,61 |
| 1926 | 0,03 | 0,10 | 3,52 | 13,09 | 0,04 | 0,69 | 2,89 | 0,07 | 0,58 | 0,05 | 78,35 | 0,00 | 0,60 |
| 1954 | 0,03 | 0,10 | 3,52 | 13,08 | 0,04 | 0,69 | 2,90 | 0,06 | 0,57 | 0,05 | 78,37 | 0,00 | 0,61 |
| 1961 | 0,03 | 0,10 | 3,53 | 13,11 | 0,04 | 0,68 | 2,90 | 0,06 | 0,59 | 0,05 | 78,30 | 0,00 | 0,61 |
| 1974 | 0,03 | 0,10 | 3,50 | 13,03 | 0,04 | 0,69 | 2,89 | 0,07 | 0,58 | 0,05 | 78,41 | 0,00 | 0,61 |
| 1998 | 0,03 | 0,10 | 3,50 | 13,01 | 0,04 | 0,68 | 2,89 | 0,07 | 0,58 | 0,05 | 78,44 | 0,00 | 0,60 |
| 2022 | 0,03 | 0,10 | 3,50 | 13,03 | 0,04 | 0,69 | 2,89 | 0,07 | 0,58 | 0,05 | 78,41 | 0,00 | 0,61 |
| 2093 | 0,03 | 0,10 | 3,51 | 13,15 | 0,04 | 0,69 | 2,89 | 0,07 | 0,60 | 0,05 | 78,26 | 0,00 | 0,61 |
| 2117 | 0,03 | 0,10 | 3,51 | 13,19 | 0,04 | 0,71 | 2,89 | 0,07 | 0,59 | 0,05 | 78,22 | 0,00 | 0,60 |
| 2141 | 0,03 | 0,09 | 3,50 | 13,11 | 0,04 | 0,63 | 2,90 | 0,07 | 0,54 | 0,05 | 78,44 | 0,00 | 0,62 |
| 2165 | 0,03 | 0,09 | 3,50 | 13,08 | 0,03 | 0,64 | 2,90 | 0,08 | 0,54 | 0,04 | 78,45 | 0,00 | 0,62 |
| 2189 | 0,03 | 0,10 | 3,54 | 13,19 | 0,04 | 0,67 | 2,89 | 0,09 | 0,56 | 0,05 | 78,21 | 0,00 | 0,64 |
| 2261 | 0,03 | 0,11 | 3,37 | 13,08 | 0,04 | 0,78 | 2,86 | 0,08 | 0,64 | 0,05 | 78,33 | 0,00 | 0,63 |
| 2285 | 0,03 | 0,11 | 3,38 | 13,11 | 0,04 | 0,76 | 2,86 | 0,08 | 0,64 | 0,05 | 78,31 | 0,00 | 0,64 |
| 2309 | 0,03 | 0,11 | 3,33 | 13,11 | 0,04 | 0,74 | 2,85 | 0,08 | 0,63 | 0,05 | 78,39 | 0,00 | 0,64 |
| 2333 | 0,03 | 0,11 | 3,34 | 13,15 | 0,04 | 0,75 | 2,85 | 0,07 | 0,65 | 0,05 | 78,33 | 0,00 | 0,64 |
| 2358 | 0,03 | 0,11 | 3,33 | 13,13 | 0,04 | 0,76 | 2,85 | 0,07 | 0,64 | 0,05 | 78,35 | 0,00 | 0,64 |
| 2429 | 0,03 | 0,11 | 3,31 | 13,00 | 0,04 | 0,76 | 2,84 | 0,08 | 0,66 | 0,05 | 78,49 | 0,00 | 0,63 |
| 2453 | 0,03 | 0,11 | 3,31 | 13,03 | 0,04 | 0,77 | 2,85 | 0,07 | 0,65 | 0,05 | 78,45 | 0,00 | 0,64 |
| 2477 | 0,03 | 0,11 | 3,30 | 12,98 | 0,04 | 0,76 | 2,85 | 0,07 | 0,65 | 0,05 | 78,50 | 0,00 | 0,65 |
| 2501 | 0,03 | 0,11 | 3,30 | 12,97 | 0,04 | 0,76 | 2,85 | 0,07 | 0,66 | 0,05 | 78,52 | 0,00 | 0,64 |
| 2525 | 0,03 | 0,11 | 3,27 | 12,90 | 0,04 | 0,76 | 2,85 | 0,08 | 0,65 | 0,05 | 78,62 | 0,00 | 0,64 |
| 2597 | 0,03 | 0,11 | 3,29 | 13,04 | 0,05 | 0,79 | 2,84 | 0,07 | 0,68 | 0,05 | 78,41 | 0,00 | 0,64 |
| 2621 | 0,03 | 0,11 | 3,30 | 13,06 | 0,04 | 0,79 | 2,84 | 0,07 | 0,68 | 0,05 | 78,38 | 0,00 | 0,64 |
| 2645 | 0,03 | 0,11 | 3,29 | 13,07 | 0,05 | 0,79 | 2,84 | 0,07 | 0,68 | 0,05 | 78,37 | 0,00 | 0,64 |
| 2669 | 0,03 | 0,11 | 3,30 | 13,09 | 0,05 | 0,78 | 2,84 | 0,07 | 0,68 | 0,05 | 78,34 | 0,00 | 0,65 |
| 2696 | 0,03 | 0,11 | 3,29 | 13,07 | 0,05 | 0,78 | 2,83 | 0,08 | 0,68 | 0,05 | 78,38 | 0,00 | 0,64 |
| 2716 | 0,03 | 0,11 | 3,30 | 13,15 | 0,04 | 0,78 | 2,83 | 0,07 | 0,67 | 0,05 | 78,32 | 0,00 | 0,63 |
| 2740 | 0,03 | 0,11 | 3,30 | 13,11 | 0,05 | 0,80 | 2,83 | 0,07 | 0,69 | 0,05 | 78,33 | 0,00 | 0,63 |
| 2764 | 0,03 | 0,11 | 3,30 | 13,13 | 0,05 | 0,81 | 2,83 | 0,07 | 0,69 | 0,05 | 78,29 | 0,00 | 0,63 |
| 2788 | 0,03 | 0,12 | 3,30 | 13,11 | 0,05 | 0,80 | 2,84 | 0,07 | 0,69 | 0,05 | 78,31 | 0,00 | 0,63 |
| 2860 | 0,03 | 0,12 | 3,29 | 13,02 | 0,05 | 0,82 | 2,84 | 0,07 | 0,70 | 0,05 | 78,38 | 0,00 | 0,63 |
| 2884 | 0,03 | 0,12 | 3,30 | 13,05 | 0,05 | 0,82 | 2,84 | 0,07 | 0,72 | 0,05 | 78,32 | 0,00 | 0,63 |
| 2908 | 0,03 | 0,12 | 3,30 | 13,12 | 0,05 | 0,82 | 2,83 | 0,07 | 0,71 | 0,05 | 78,26 | 0,00 | 0,63 |
| 2932 | 0,03 | 0,12 | 3,31 | 13,16 | 0,05 | 0,84 | 2,83 | 0,07 | 0,72 | 0,06 | 78,19 | 0,00 | 0,63 |
| 2956 | 0,03 | 0,12 | 3,30 | 13,13 | 0,05 | 0,83 | 2,84 | 0,07 | 0,72 | 0,05 | 78,24 | 0,00 | 0,62 |
| 3028 | 0,03 | 0,12 | 3,29 | 13,34 | 0,05 | 0,81 | 2,84 | 0,08 | 0,71 | 0,05 | 78,06 | 0,00 | 0,62 |
| 3076 | 0,03 | 0,12 | 3,29 | 13,43 | 0,04 | 0,83 | 2,84 | 0,07 | 0,72 | 0,06 | 77,87 | 0,06 | 0,64 |
| 3100 | 0,03 | 0,13 | 3,28 | 13,45 | 0,04 | 0,86 | 2,83 | 0,07 | 0,74 | 0,06 | 77,83 | 0,08 | 0,61 |
| 3124 | 0,03 | 0,13 | 3,28 | 13,45 | 0,04 | 0,88 | 2,83 | 0,07 | 0,77 | 0,06 | 77,75 | 0,08 | 0,63 |
| 3196 | 0,03 | 0,12 | 3,25 | 13,12 | 0,04 | 0,88 | 2,85 | 0,07 | 0,77 | 0,06 | 78,09 | 0,09 | 0,61 |
| 3220 | 0,03 | 0,13 | 3,25 | 13,13 | 0,04 | 0,88 | 2,85 | 0,07 | 0,78 | 0,06 | 78,10 | 0,09 | 0,59 |
| 3244 | 0,03 | 0,13 | 3,23 | 13,15 | 0,04 | 0,89 | 2,85 | 0,07 | 0,78 | 0,06 | 78,18 | 0,00 | 0,59 |
| 3268 | 0,03 | 0,13 | 3,23 | 13,17 | 0,05 | 0,91 | 2,86 | 0,07 | 0,79 | 0,06 | 78,03 | 0,09 | 0,58 |
| 3292 | 0,03 | 0,13 | 3,23 | 13,15 | 0,05 | 0,91 | 2,86 | 0,07 | 0,79 | 0,06 | 78,03 | 0,08 | 0,60 |
| 3364 | 0,03 | 0,08 | 3,10 | 7,81 | 0,05 | 0,94 | 3,06 | 0,06 | 0,85 | 0,06 | 83,23 | 0,08 | 0,64 |
| 3388 | 0,03 | 0,07 | 3,11 | 7,82 | 0,05 | 0,94 | 3,06 | 0,06 | 0,85 | 0,06 | 83,23 | 0,09 | 0,62 |
| 3412 | 0,03 | 0,09 | 3,11 | 8,89 | 0,05 | 0,94 | 3,02 | 0,06 | 0,85 | 0,06 | 82,19 | 0,08 | 0,62 |
| 3436 | 0,03 | 0,09 | 3,13 | 9,17 | 0,05 | 0,95 | 3,01 | 0,06 | 0,85 | 0,06 | 81,89 | 0,09 | 0,62 |
| 3555 | 0,03 | 0,11 | 3,16 | 11,17 | 0,05 | 0,96 | 2,91 | 0,07 | 0,85 | 0,06 | 80,00 | 0,00 | 0,62 |
| 3580 | 0,03 | 0,04 | 3,03 | 5,09 | 0,05 | 1,01 | 3,15 | 0,06 | 0,95 | 0,07 | 85,88 | 0,00 | 0,63 |
| 3604 | 0,03 | 0,04 | 3,01 | 4,17 | 0,05 | 1,00 | 3,18 | 0,05 | 0,93 | 0,07 | 86,82 | 0,00 | 0,65 |
| 3628 | 0,03 | 0,04 | 3,00 | 4,07 | 0,05 | 1,00 | 3,19 | 0,05 | 0,93 | 0,07 | 86,90 | 0,00 | 0,67 |
| 3700 | 0,04 | 0,17 | 3,27 | 15,95 | 0,05 | 0,96 | 2,76 | 0,08 | 0,84 | 0,06 | 75,18 | 0,08 | 0,57 |
| 3724 | 0,04 | 0,18 | 3,27 | 15,99 | 0,05 | 0,96 | 2,75 | 0,08 | 0,85 | 0,06 | 75,14 | 0,08 | 0,56 |
| 3748 | 0,04 | 0,12 | 3,19 | 12,16 | 0,05 | 1,00 | 2,90 | 0,07 | 0,90 | 0,07 | 78,84 | 0,08 | 0,58 |
| 3772 | 0,04 | 0,12 | 3,17 | 11,30 | 0,05 | 0,99 | 2,93 | 0,07 | 0,90 | 0,07 | 79,70 | 0,09 | 0,58 |
| 3796 | 0,04 | 0,12 | 3,16 | 11,22 | 0,05 | 1,00 | 2,93 | 0,07 | 0,90 | 0,07 | 79,77 | 0,09 | 0,59 |
| 3868 | 0,04 | 0,15 | 3,22 | 13,58 | 0,05 | 1,00 | 2,84 | 0,08 | 0,89 | 0,07 | 77,41 | 0,08 | 0,58 |
| 3892 | 0,04 | 0,15 | 3,22 | 13,60 | 0,05 | 1,00 | 2,84 | 0,08 | 0,90 | 0,07 | 77,41 | 0,09 | 0,57 |
| 4016 | 0,02 | 0,09 | 3,22 | 13,47 | 0,04 | 0,63 | 2,88 | 0,10 | 0,51 | 0,03 | 78,41 | 0,00 | 0,60 |

### Beispiel 9 (nicht erfindungsgemäß)

Der in Beispiel 8 beschriebene Versuch wurde analog mit demselben Edukt und unter denselben Bedingungen durchgeführt. Es wurde jedoch ein chromhaltiger Katalysator eingesetzt, wie er auch für die in DE19842370A1 beschrieben Versuche verwendet wurde. Der zeitliche Verlauf der Zusammensetzung des Produktgemisches ist in Tabelle 11 wiedergegen; die Zusammensetzung des Eduktgemisches entspricht dabei dem Tabelleneintrag zum Zeitpunkt t=0.

**Tabelle 11: Zeitlicher Verlauf der Zusammensetzung des Produktgemisches gemäß Beispiel 9**

| t [h] | 2-Methylbutanal | n-Pentanal | 2-Me-1-Butanol | n-Pentanol | 4-Me-2-Pr-2-Hexenal | 4-Me-2-Pr-2-Hexanal | 4-Me-2-Pr-Hexenol | 4-Me-2-Pr-Hexanol | 2-Pr-2-Heptenal (1) linear | 2-Pr-2-Heptenal (2) verzweigt | 2-PH-ol | 2-PHE-ol | Unbekannte |
|---|---|---|---|---|---|---|---|---|---|---|---|---|---|
| 0 | 1,64 | 13,15 | 0,01 | 0,16 | 1,91 | 1,00 | 0,00 | 0,00 | 0,00 | 81,66 | 0,00 | 0,00 | 0,47 |
| 18 | 0,02 | 0,13 | 1,71 | 13,23 | 0,05 | 0,71 | 1,71 | 0,09 | 0,75 | 0,07 | 80,96 | 0,08 | 0,49 |
| 26 | 0,01 | 0,11 | 1,72 | 13,31 | 0,05 | 0,71 | 1,71 | 0,09 | 0,58 | 0,05 | 81,10 | 0,05 | 0,51 |
| 42 | 0,02 | 0,13 | 1,71 | 13,32 | 0,05 | 0,72 | 1,71 | 0,09 | 0,71 | 0,06 | 80,92 | 0,00 | 0,56 |
| 50 | 0,01 | 0,10 | 1,72 | 13,35 | 0,04 | 0,73 | 1,71 | 0,09 | 0,54 | 0,04 | 81,08 | 0,00 | 0,57 |
| 66 | 0,02 | 0,11 | 1,72 | 13,33 | 0,04 | 0,75 | 1,71 | 0,09 | 0,60 | 0,05 | 80,99 | 0,00 | 0,58 |
| 73 | 0,01 | 0,11 | 1,72 | 13,34 | 0,04 | 0,76 | 1,71 | 0,09 | 0,53 | 0,04 | 81,06 | 0,00 | 0,59 |
| 138 | 0,02 | 0,11 | 1,72 | 13,33 | 0,01 | 0,82 | 1,71 | 0,09 | 0,58 | 0,04 | 80,95 | 0,00 | 0,63 |
| 146 | 0,02 | 0,11 | 1,71 | 13,32 | 0,04 | 0,82 | 1,71 | 0,09 | 0,57 | 0,04 | 80,96 | 0,00 | 0,59 |
| 163 | 0,02 | 0,11 | 1,72 | 13,41 | 0,01 | 0,83 | 1,71 | 0,09 | 0,57 | 0,04 | 80,86 | 0,00 | 0,63 |
| 170 | 0,02 | 0,12 | 1,72 | 13,42 | 0,01 | 0,84 | 1,71 | 0,09 | 0,59 | 0,04 | 80,83 | 0,00 | 0,62 |
| 187 | 0,02 | 0,12 | 1,72 | 13,44 | 0,04 | 0,86 | 1,71 | 0,09 | 0,59 | 0,04 | 80,80 | 0,00 | 0,58 |
| 194 | 0,02 | 0,12 | 1,72 | 13,41 | 0,04 | 0,86 | 1,71 | 0,08 | 0,68 | 0,06 | 80,72 | 0,00 | 0,58 |
| 210 | 0,02 | 0,12 | 1,72 | 13,42 | 0,03 | 0,87 | 1,71 | 0,08 | 0,61 | 0,05 | 80,79 | 0,00 | 0,58 |
| 217 | 0,02 | 0,11 | 1,72 | 13,43 | 0,04 | 0,87 | 1,71 | 0,08 | 0,59 | 0,05 | 80,80 | 0,00 | 0,59 |
| 238 | 0,02 | 0,12 | 1,71 | 13,40 | 0,03 | 0,87 | 1,71 | 0,08 | 0,61 | 0,05 | 80,81 | 0,00 | 0,59 |
| 242 | 0,02 | 0,12 | 1,72 | 13,42 | 0,04 | 0,88 | 1,71 | 0,08 | 0,60 | 0,05 | 80,78 | 0,00 | 0,59 |
| 306 | 0,02 | 0,12 | 1,72 | 13,44 | 0,03 | 0,92 | 1,70 | 0,08 | 0,64 | 0,05 | 80,70 | 0,00 | 0,58 |
| 331 | 0,02 | 0,13 | 1,70 | 12,49 | 0,04 | 1,07 | 1,72 | 0,10 | 0,75 | 0,05 | 81,31 | 0,05 | 0,57 |
| 338 | 0,02 | 0,13 | 1,70 | 12,56 | 0,04 | 1,07 | 1,72 | 0,09 | 0,74 | 0,05 | 81,28 | 0,04 | 0,56 |
| 354 | 0,02 | 0,13 | 1,71 | 12,62 | 0,04 | 1,07 | 1,72 | 0,08 | 0,73 | 0,05 | 81,24 | 0,04 | 0,55 |
| 361 | 0,02 | 0,13 | 1,70 | 12,64 | 0,04 | 1,08 | 1,72 | 0,08 | 0,72 | 0,05 | 81,23 | 0,04 | 0,55 |
| 379 | 0,02 | 0,14 | 1,72 | 13,77 | 0,04 | 1,08 | 1,69 | 0,08 | 0,71 | 0,05 | 80,16 | 0,00 | 0,54 |
| 385 | 0,02 | 0,14 | 1,72 | 13,89 | 0,04 | 1,08 | 1,69 | 0,13 | 0,71 | 0,05 | 79,95 | 0,04 | 0,54 |
| 450 | 0,02 | 0,15 | 1,72 | 14,07 | 0,04 | 1,11 | 1,68 | 0,08 | 0,70 | 0,05 | 79,85 | 0,00 | 0,53 |
| 458 | 0,02 | 0,14 | 1,72 | 14,09 | 0,04 | 1,10 | 1,68 | 0,08 | 0,69 | 0,05 | 79,86 | 0,00 | 0,53 |
| 498 | 0,02 | 0,14 | 1,72 | 13,50 | 0,04 | 1,17 | 1,69 | 0,08 | 0,73 | 0,05 | 80,32 | 0,00 | 0,54 |
| 522 | 0,02 | 0,14 | 1,72 | 13,44 | 0,04 | 1,18 | 1,70 | 0,08 | 0,73 | 0,05 | 80,36 | 0,00 | 0,53 |
| 530 | 0,02 | 0,15 | 1,71 | 13,41 | 0,04 | 1,18 | 1,69 | 0,08 | 0,81 | 0,06 | 80,29 | 0,00 | 0,54 |
| 619 | 0,02 | 0,15 | 1,71 | 13,34 | 0,04 | 1,21 | 1,69 | 0,08 | 0,74 | 0,06 | 80,43 | 0,00 | 0,53 |
| 626 | 0,02 | 0,14 | 1,71 | 13,34 | 0,04 | 1,21 | 1,69 | 0,08 | 0,74 | 0,06 | 80,44 | 0,00 | 0,53 |
| 643 | 0,02 | 0,15 | 1,71 | 13,34 | 0,04 | 1,22 | 1,69 | 0,08 | 0,76 | 0,06 | 80,40 | 0,00 | 0,53 |
| 660 | 0,03 | 0,14 | 2,58 | 13,20 | 0,06 | 1,15 | 2,68 | 0,09 | 0,70 | 0,05 | 78,75 | 0,00 | 0,56 |
| 667 | 0,03 | 0,14 | 2,76 | 13,18 | 0,07 | 1,16 | 2,90 | 0,08 | 0,72 | 0,06 | 78,28 | 0,04 | 0,56 |
| 684 | 0,03 | 0,14 | 2,89 | 13,15 | 0,07 | 1,19 | 3,04 | 0,08 | 0,72 | 0,06 | 78,03 | 0,04 | 0,55 |
| 708 | 0,04 | 0,16 | 3,00 | 14,02 | 0,07 | 1,19 | 3,13 | 0,08 | 0,72 | 0,06 | 76,95 | 0,05 | 0,55 |
| 715 | 0,04 | 0,16 | 3,04 | 14,23 | 0,07 | 1,22 | 3,18 | 0,08 | 0,72 | 0,05 | 76,63 | 0,05 | 0,54 |
| 780 | 0,04 | 0,16 | 3,13 | 14,41 | 0,07 | 1,27 | 3,27 | 0,08 | 0,72 | 0,05 | 76,21 | 0,05 | 0,52 |
| 804 | 0,04 | 0,12 | 3,27 | 11,33 | 0,08 | 1,33 | 3,54 | 0,07 | 0,80 | 0,06 | 78,77 | 0,05 | 0,53 |
| 811 | 0,04 | 0,12 | 3,31 | 10,50 | 0,08 | 1,34 | 3,61 | 0,06 | 0,79 | 0,06 | 79,51 | 0,05 | 0,53 |
| 828 | 0,04 | 0,11 | 3,34 | 9,88 | 0,08 | 1,35 | 3,67 | 0,06 | 0,79 | 0,06 | 80,03 | 0,04 | 0,54 |
| 835 | 0,04 | 0,11 | 3,35 | 9,82 | 0,08 | 1,35 | 3,67 | 0,06 | 0,80 | 0,06 | 80,05 | 0,05 | 0,54 |
| 852 | 0,04 | 0,12 | 3,34 | 9,79 | 0,08 | 1,36 | 3,68 | 0,06 | 0,80 | 0,06 | 80,08 | 0,05 | 0,54 |
| 859 | 0,04 | 0,12 | 3,34 | 9,82 | 0,08 | 1,37 | 3,67 | 0,06 | 0,78 | 0,06 | 80,07 | 0,05 | 0,55 |
| 876 | 0,04 | 0,20 | 3,27 | 15,20 | 0,08 | 1,30 | 3,39 | 0,07 | 0,74 | 0,06 | 75,08 | 0,05 | 0,52 |
| 883 | 0,04 | 0,20 | 3,27 | 15,81 | 0,08 | 1,30 | 3,38 | 0,07 | 0,75 | 0,06 | 74,49 | 0,05 | 0,51 |
| 972 | 0,05 | 0,20 | 3,28 | 16,33 | 0,08 | 1,33 | 3,36 | 0,08 | 0,76 | 0,06 | 73,94 | 0,05 | 0,49 |
| 996 | 0,05 | 0,17 | 3,54 | 14,47 | 0,08 | 1,37 | 3,47 | 0,07 | 0,78 | 0,06 | 75,39 | 0,05 | 0,50 |
| 1004 | 0,05 | 0,17 | 3,62 | 13,86 | 0,08 | 1,38 | 3,50 | 0,07 | 0,77 | 0,06 | 75,89 | 0,05 | 0,50 |
| 1020 | 0,05 | 0,17 | 3,67 | 13,55 | 0,08 | 1,40 | 3,52 | 0,07 | 0,79 | 0,06 | 76,09 | 0,04 | 0,51 |
| 1116 | 0,05 | 0,17 | 3,72 | 13,05 | 0,08 | 1,44 | 3,54 | 0,07 | 0,80 | 0,06 | 76,45 | 0,06 | 0,50 |
| 1124 | 0,05 | 0,17 | 3,72 | 13,08 | 0,08 | 1,45 | 3,54 | 0,07 | 0,81 | 0,06 | 76,41 | 0,05 | 0,51 |
| 1140 | 0,05 | 0,18 | 3,67 | 13,42 | 0,08 | 1,44 | 3,52 | 0,07 | 0,82 | 0,06 | 76,13 | 0,05 | 0,50 |
| 1148 | 0,05 | 0,17 | 3,65 | 13,48 | 0,08 | 1,45 | 3,52 | 0,07 | 0,82 | 0,06 | 76,08 | 0,05 | 0,50 |
| 1164 | 0,05 | 0,17 | 3,71 | 13,16 | 0,08 | 1,48 | 3,54 | 0,07 | 0,80 | 0,06 | 76,33 | 0,05 | 0,50 |
| 1171 | 0,05 | 0,17 | 3,72 | 13,13 | 0,09 | 1,49 | 3,54 | 0,07 | 0,84 | 0,07 | 76,28 | 0,05 | 0,50 |
| 1188 | 0,06 | 0,17 | 3,72 | 13,08 | 0,09 | 1,50 | 3,54 | 0,07 | 0,82 | 0,06 | 76,34 | 0,05 | 0,50 |
| 1212 | 0,05 | 0,17 | 3,73 | 12,99 | 0,08 | 1,51 | 3,54 | 0,07 | 0,81 | 0,06 | 76,42 | 0,05 | 0,50 |
| 1219 | 0,05 | 0,17 | 3,74 | 12,99 | 0,09 | 1,52 | 3,55 | 0,07 | 0,82 | 0,06 | 76,41 | 0,05 | 0,50 |
| 1284 | 0,06 | 0,18 | 3,73 | 12,96 | 0,09 | 1,55 | 3,54 | 0,07 | 0,83 | 0,06 | 76,39 | 0,05 | 0,50 |
| 1292 | 0,05 | 0,17 | 3,74 | 12,97 | 0,08 | 1,53 | 3,54 | 0,07 | 0,81 | 0,06 | 76,42 | 0,05 | 0,50 |
| 1308 | 0,06 | 0,18 | 3,73 | 12,96 | 0,09 | 1,55 | 3,54 | 0,06 | 0,84 | 0,06 | 76,38 | 0,05 | 0,51 |
| 1315 | 0,06 | 0,18 | 3,73 | 12,87 | 0,09 | 1,55 | 3,54 | 0,07 | 0,84 | 0,06 | 76,46 | 0,05 | 0,50 |
| 1332 | 0,06 | 0,18 | 3,73 | 12,69 | 0,09 | 1,57 | 3,56 | 0,07 | 0,89 | 0,08 | 76,54 | 0,05 | 0,50 |
| 1339 | 0,06 | 0,17 | 3,74 | 12,70 | 0,09 | 1,58 | 3,56 | 0,06 | 0,83 | 0,06 | 76,60 | 0,05 | 0,50 |
| 1356 | 0,06 | 0,18 | 3,74 | 12,68 | 0,09 | 1,59 | 3,56 | 0,06 | 0,85 | 0,06 | 76,59 | 0,05 | 0,50 |
| 1363 | 0,06 | 0,17 | 3,73 | 12,66 | 0,09 | 1,58 | 3,56 | 0,06 | 0,82 | 0,06 | 76,65 | 0,05 | 0,50 |
| 1380 | 0,06 | 0,17 | 3,73 | 12,65 | 0,09 | 1,59 | 3,56 | 0,06 | 0,86 | 0,07 | 76,61 | 0,05 | 0,50 |
| 1452 | 0,05 | 0,18 | 3,57 | 12,95 | 0,08 | 1,61 | 3,07 | 0,08 | 0,86 | 0,07 | 76,88 | 0,05 | 0,57 |
| 1460 | 0,06 | 0,18 | 3,58 | 12,97 | 0,08 | 1,62 | 3,07 | 0,08 | 0,85 | 0,07 | 76,85 | 0,05 | 0,57 |
| 1476 | 0,06 | 0,18 | 3,57 | 12,95 | 0,08 | 1,61 | 3,06 | 0,08 | 0,87 | 0,07 | 76,85 | 0,05 | 0,56 |
| 1483 | 0,06 | 0,18 | 3,58 | 12,96 | 0,08 | 1,62 | 3,06 | 0,08 | 0,87 | 0,07 | 76,83 | 0,05 | 0,56 |
| 1500 | 0,06 | 0,18 | 3,57 | 12,96 | 0,08 | 1,63 | 3,06 | 0,08 | 0,87 | 0,07 | 76,84 | 0,05 | 0,55 |
| 1520 | 0,06 | 0,19 | 3,54 | 13,02 | 0,08 | 1,66 | 2,95 | 0,08 | 0,88 | 0,07 | 76,85 | 0,05 | 0,58 |
| 1528 | 0,06 | 0,19 | 3,53 | 13,03 | 0,08 | 1,66 | 2,93 | 0,08 | 0,89 | 0,07 | 76,84 | 0,05 | 0,58 |
| 1546 | 0,06 | 0,19 | 3,53 | 13,03 | 0,07 | 1,67 | 2,93 | 0,08 | 0,88 | 0,07 | 76,85 | 0,05 | 0,58 |
| 1616 | 0,06 | 0,19 | 3,51 | 13,12 | 0,08 | 1,70 | 2,86 | 0,09 | 0,90 | 0,07 | 76,80 | 0,06 | 0,58 |
| 1624 | 0,06 | 0,19 | 3,51 | 13,11 | 0,08 | 1,71 | 2,86 | 0,09 | 0,90 | 0,07 | 76,79 | 0,06 | 0,59 |
| 1640 | 0,06 | 0,20 | 3,50 | 13,10 | 0,08 | 1,72 | 2,86 | 0,09 | 0,92 | 0,07 | 76,77 | 0,06 | 0,58 |
| 1648 | 0,06 | 0,19 | 3,50 | 13,13 | 0,07 | 1,72 | 2,85 | 0,09 | 0,87 | 0,07 | 76,82 | 0,06 | 0,58 |
| 1665 | 0,06 | 0,20 | 3,50 | 13,18 | 0,07 | 1,72 | 2,83 | 0,08 | 0,91 | 0,07 | 76,73 | 0,05 | 0,59 |
| 1672 | 0,06 | 0,20 | 3,50 | 13,20 | 0,07 | 1,73 | 2,83 | 0,09 | 0,90 | 0,07 | 76,71 | 0,05 | 0,59 |
| 1688 | 0,06 | 0,20 | 3,50 | 13,19 | 0,08 | 1,74 | 2,82 | 0,09 | 0,92 | 0,07 | 76,69 | 0,06 | 0,59 |
| 1717 | 0,06 | 0,19 | 3,48 | 13,09 | 0,07 | 1,74 | 2,82 | 0,09 | 0,91 | 0,07 | 76,84 | 0,06 | 0,58 |
| 1724 | 0,06 | 0,20 | 3,49 | 13,12 | 0,08 | 1,75 | 2,82 | 0,09 | 0,95 | 0,09 | 76,71 | 0,05 | 0,59 |
| 1784 | 0,06 | 0,20 | 3,49 | 13,14 | 0,08 | 1,78 | 2,81 | 0,09 | 0,95 | 0,07 | 76,67 | 0,06 | 0,58 |
| 1808 | 0,06 | 0,20 | 3,49 | 13,09 | 0,08 | 1,79 | 2,81 | 0,09 | 0,95 | 0,07 | 76,73 | 0,06 | 0,58 |
| 1820 | 0,06 | 0,20 | 3,50 | 13,10 | 0,08 | 1,80 | 2,81 | 0,09 | 0,92 | 0,07 | 76,74 | 0,06 | 0,58 |
| 1832 | 0,06 | 0,21 | 3,49 | 13,08 | 0,08 | 1,81 | 2,81 | 0,09 | 0,97 | 0,07 | 76,70 | 0,06 | 0,58 |
| 1840 | 0,06 | 0,20 | 3,49 | 13,07 | 0,08 | 1,81 | 2,82 | 0,09 | 0,94 | 0,07 | 76,73 | 0,06 | 0,58 |
| 1856 | 0,06 | 0,20 | 3,49 | 13,07 | 0,08 | 1,82 | 2,80 | 0,08 | 0,94 | 0,07 | 76,74 | 0,06 | 0,58 |
| 1880 | 0,06 | 0,20 | 3,48 | 13,05 | 0,07 | 1,82 | 2,79 | 0,09 | 0,96 | 0,07 | 76,75 | 0,06 | 0,60 |
| 1891 | 0,06 | 0,21 | 3,48 | 13,05 | 0,08 | 1,82 | 2,80 | 0,09 | 0,99 | 0,08 | 76,71 | 0,07 | 0,57 |
| 1952 | 0,06 | 0,21 | 3,49 | 13,06 | 0,08 | 1,87 | 2,80 | 0,09 | 0,98 | 0,08 | 76,65 | 0,07 | 0,58 |
| 1981 | 0,06 | 0,21 | 3,48 | 13,05 | 0,08 | 1,88 | 2,80 | 0,09 | 0,99 | 0,08 | 76,65 | 0,06 | 0,57 |
| 1988 | 0,06 | 0,21 | 3,49 | 13,04 | 0,08 | 1,89 | 2,81 | 0,09 | 1,00 | 0,08 | 76,61 | 0,07 | 0,58 |
| 2001 | 0,06 | 0,21 | 3,47 | 12,99 | 0,08 | 1,88 | 2,78 | 0,09 | 0,99 | 0,08 | 76,72 | 0,07 | 0,58 |
| 2024 | 0,06 | 0,21 | 3,49 | 13,04 | 0,08 | 1,91 | 2,80 | 0,08 | 0,97 | 0,07 | 76,64 | 0,06 | 0,58 |
| 2048 | 0,06 | 0,21 | 3,47 | 13,01 | 0,08 | 1,92 | 2,80 | 0,09 | 1,01 | 0,08 | 76,62 | 0,07 | 0,57 |
| 2120 | 0,06 | 0,22 | 3,48 | 13,05 | 0,08 | 1,96 | 2,80 | 0,09 | 1,04 | 0,08 | 76,52 | 0,07 | 0,57 |
| 2144 | 0,07 | 0,22 | 3,48 | 13,04 | 0,08 | 1,97 | 2,80 | 0,09 | 1,03 | 0,08 | 76,51 | 0,07 | 0,57 |
| 2168 | 0,06 | 0,21 | 3,47 | 13,17 | 0,08 | 1,89 | 2,83 | 0,09 | 0,99 | 0,08 | 76,49 | 0,07 | 0,57 |
| 2192 | 0,06 | 0,21 | 3,48 | 13,10 | 0,08 | 1,92 | 2,81 | 0,09 | 1,01 | 0,08 | 76,51 | 0,07 | 0,57 |
| 2216 | 0,06 | 0,21 | 3,47 | 13,00 | 0,08 | 1,93 | 2,80 | 0,09 | 1,00 | 0,08 | 76,61 | 0,07 | 0,58 |
| 2288 | 0,07 | 0,22 | 3,36 | 13,03 | 0,07 | 2,01 | 2,76 | 0,10 | 1,08 | 0,08 | 76,55 | 0,08 | 0,59 |
| 2312 | 0,06 | 0,22 | 3,36 | 13,04 | 0,09 | 2,01 | 2,77 | 0,10 | 1,06 | 0,07 | 76,55 | 0,07 | 0,60 |
| 2315 | 0,06 | 0,22 | 3,35 | 13,01 | 0,08 | 1,91 | 2,79 | 0,13 | 1,02 | 0,08 | 76,62 | 0,06 | 0,67 |
| 2331 | 0,06 | 0,22 | 3,32 | 13,04 | 0,08 | 2,01 | 2,76 | 0,10 | 1,05 | 0,08 | 76,59 | 0,07 | 0,61 |
| 2355 | 0,06 | 0,22 | 3,31 | 13,06 | 0,08 | 2,03 | 2,75 | 0,10 | 1,06 | 0,08 | 76,56 | 0,07 | 0,60 |
| 2379 | 0,06 | 0,22 | 3,31 | 13,05 | 0,08 | 2,03 | 2,75 | 0,10 | 1,07 | 0,09 | 76,51 | 0,07 | 0,64 |
| 2451 | 0,06 | 0,22 | 3,27 | 12,95 | 0,08 | 2,08 | 2,75 | 0,10 | 1,08 | 0,08 | 76,64 | 0,07 | 0,61 |
| 2475 | 0,07 | 0,23 | 3,28 | 12,96 | 0,08 | 2,10 | 2,75 | 0,10 | 1,08 | 0,08 | 76,60 | 0,07 | 0,61 |
| 2499 | 0,06 | 0,22 | 3,27 | 12,93 | 0,08 | 2,10 | 2,74 | 0,10 | 1,07 | 0,08 | 76,65 | 0,07 | 0,61 |
| 2523 | 0,07 | 0,23 | 3,26 | 12,89 | 0,09 | 2,12 | 2,74 | 0,10 | 1,09 | 0,08 | 76,65 | 0,07 | 0,61 |
| 2547 | 0,07 | 0,23 | 3,27 | 12,91 | 0,08 | 2,14 | 2,74 | 0,10 | 1,10 | 0,08 | 76,60 | 0,07 | 0,61 |
| 2619 | 0,07 | 0,23 | 3,26 | 12,97 | 0,08 | 2,16 | 2,73 | 0,10 | 1,10 | 0,08 | 76,54 | 0,08 | 0,61 |
| 2643 | 0,07 | 0,24 | 3,26 | 12,96 | 0,08 | 2,18 | 2,73 | 0,10 | 1,14 | 0,09 | 76,47 | 0,08 | 0,61 |
| 2667 | 0,07 | 0,24 | 3,25 | 12,97 | 0,09 | 2,20 | 2,73 | 0,10 | 1,14 | 0,09 | 76,43 | 0,08 | 0,61 |
| 2691 | 0,07 | 0,23 | 3,26 | 13,00 | 0,09 | 2,21 | 2,73 | 0,10 | 1,09 | 0,08 | 76,45 | 0,08 | 0,61 |
| 2716 | 0,07 | 0,24 | 3,26 | 12,98 | 0,08 | 2,23 | 2,73 | 0,10 | 1,15 | 0,09 | 76,39 | 0,08 | 0,61 |
| 2787 | 0,07 | 0,24 | 3,25 | 13,02 | 0,08 | 2,25 | 2,72 | 0,10 | 1,16 | 0,09 | 76,32 | 0,08 | 0,61 |
| 2811 | 0,07 | 0,25 | 3,26 | 13,02 | 0,08 | 2,26 | 2,73 | 0,10 | 1,16 | 0,09 | 76,31 | 0,08 | 0,61 |
| 2835 | 0,07 | 0,24 | 3,25 | 13,02 | 0,08 | 2,27 | 2,72 | 0,10 | 1,16 | 0,09 | 76,30 | 0,08 | 0,61 |
| 2859 | 0,07 | 0,25 | 3,26 | 13,04 | 0,08 | 2,29 | 2,72 | 0,10 | 1,17 | 0,09 | 76,24 | 0,08 | 0,61 |
| 2883 | 0,07 | 0,25 | 3,25 | 13,01 | 0,08 | 2,30 | 2,72 | 0,10 | 1,18 | 0,09 | 76,25 | 0,08 | 0,61 |
| 2955 | 0,07 | 0,25 | 3,25 | 13,06 | 0,08 | 2,32 | 2,71 | 0,10 | 1,22 | 0,09 | 76,14 | 0,09 | 0,61 |
| 2979 | 0,07 | 0,25 | 3,26 | 13,08 | 0,08 | 2,35 | 2,72 | 0,10 | 1,17 | 0,09 | 76,13 | 0,09 | 0,61 |
| 3003 | 0,07 | 0,25 | 3,25 | 13,06 | 0,08 | 2,36 | 2,71 | 0,10 | 1,19 | 0,09 | 76,14 | 0,09 | 0,61 |
| 3027 | 0,07 | 0,25 | 3,25 | 13,03 | 0,08 | 2,39 | 2,72 | 0,10 | 1,19 | 0,09 | 76,13 | 0,09 | 0,61 |
| 3051 | 0,07 | 0,26 | 3,23 | 12,99 | 0,08 | 2,38 | 2,71 | 0,11 | 1,22 | 0,10 | 76,15 | 0,09 | 0,60 |
| 3123 | 0,08 | 0,27 | 3,24 | 13,22 | 0,09 | 2,44 | 2,70 | 0,10 | 1,26 | 0,09 | 75,82 | 0,09 | 0,59 |
| 3171 | 0,07 | 0,27 | 3,23 | 13,29 | 0,09 | 2,44 | 2,71 | 0,10 | 1,25 | 0,09 | 75,74 | 0,14 | 0,58 |
| 3195 | 0,07 | 0,27 | 3,24 | 13,33 | 0,09 | 2,46 | 2,71 | 0,10 | 1,21 | 0,09 | 75,71 | 0,13 | 0,59 |
| 3218 | 0,07 | 0,27 | 3,23 | 13,32 | 0,09 | 2,47 | 2,70 | 0,10 | 1,26 | 0,09 | 75,65 | 0,14 | 0,59 |
| 3290 | 0,08 | 0,27 | 3,23 | 13,32 | 0,09 | 2,50 | 2,70 | 0,11 | 1,29 | 0,10 | 75,59 | 0,16 | 0,57 |
| 3314 | 0,07 | 0,27 | 3,21 | 13,23 | 0,09 | 2,53 | 2,71 | 0,10 | 1,26 | 0,09 | 75,70 | 0,14 | 0,59 |
| 3327 | 0,07 | 0,27 | 3,21 | 13,18 | 0,09 | 2,46 | 2,72 | 0,11 | 1,28 | 0,09 | 75,82 | 0,14 | 0,58 |
| 3349 | 0,08 | 0,27 | 3,19 | 13,11 | 0,09 | 2,51 | 2,73 | 0,11 | 1,28 | 0,09 | 75,83 | 0,14 | 0,58 |
| 3373 | 0,08 | 0,27 | 3,18 | 13,11 | 0,09 | 2,54 | 2,72 | 0,10 | 1,29 | 0,09 | 75,79 | 0,15 | 0,58 |
| 3445 | 0,07 | 0,18 | 3,09 | 9,04 | 0,10 | 2,68 | 2,86 | 0,08 | 1,36 | 0,10 | 79,68 | 0,15 | 0,60 |
| 3469 | 0,08 | 0,19 | 3,08 | 8,96 | 0,10 | 2,69 | 2,87 | 0,09 | 1,43 | 0,10 | 79,66 | 0,15 | 0,61 |
| 3493 | 0,08 | 0,44 | 3,25 | 16,40 | 0,09 | 2,43 | 2,60 | 0,11 | 1,19 | 0,09 | 72,47 | 0,15 | 0,72 |
| 3517 | 0,08 | 0,45 | 3,35 | 20,46 | 0,09 | 2,49 | 2,45 | 0,13 | 1,20 | 0,09 | 68,52 | 0,14 | 0,56 |
| 3637 | 0,01 | 0,44 | 3,31 | 18,83 | 0,09 | 2,67 | 2,50 | 0,13 | 1,31 | 0,09 | 69,89 | 0,15 | 0,56 |
| 3661 | 0,08 | 0,16 | 3,10 | 9,56 | 0,10 | 2,94 | 2,83 | 0,10 | 1,49 | 0,11 | 78,78 | 0,16 | 0,59 |
| 3685 | 0,08 | 0,15 | 3,03 | 6,83 | 0,11 | 2,98 | 2,94 | 0,08 | 1,56 | 0,11 | 81,36 | 0,14 | 0,62 |
| 3709 | 0,08 | 0,15 | 3,01 | 6,61 | 0,11 | 3,00 | 2,94 | 0,08 | 1,56 | 0,11 | 81,59 | 0,15 | 0,61 |
| 3781 | 0,09 | 0,45 | 3,30 | 18,89 | 0,10 | 2,80 | 2,48 | 0,13 | 1,34 | 0,10 | 69,60 | 0,16 | 0,57 |
| 3805 | 0,09 | 0,47 | 3,29 | 18,89 | 0,10 | 2,83 | 2,48 | 0,13 | 1,39 | 0,10 | 69,52 | 0,15 | 0,56 |
| 3829 | 0,09 | 0,39 | 3,24 | 16,82 | 0,10 | 2,92 | 2,55 | 0,12 | 1,44 | 0,10 | 71,50 | 0,16 | 0,56 |
| 3853 | 0,09 | 0,38 | 3,21 | 15,85 | 0,10 | 2,96 | 2,59 | 0,12 | 1,43 | 0,10 | 72,43 | 0,16 | 0,57 |
| 3877 | 0,09 | 0,39 | 3,21 | 15,76 | 0,11 | 3,00 | 2,59 | 0,12 | 1,50 | 0,11 | 72,40 | 0,16 | 0,57 |
| 3949 | 0,09 | 0,39 | 3,20 | 15,63 | 0,11 | 3,08 | 2,58 | 0,12 | 1,49 | 0,10 | 72,45 | 0,18 | 0,57 |
| 3973 | 0,10 | 0,40 | 3,20 | 15,60 | 0,11 | 3,13 | 2,59 | 0,13 | 1,51 | 0,11 | 72,38 | 0,17 | 0,58 |
| 3997 | 0,09 | 0,36 | 3,18 | 14,45 | 0,11 | 3,20 | 2,62 | 0,12 | 1,58 | 0,11 | 73,41 | 0,18 | 0,59 |
| 4117 | 0,10 | 0,37 | 3,15 | 13,73 | 0,12 | 3,43 | 2,63 | 0,11 | 1,65 | 0,11 | 73,82 | 0,19 | 0,58 |

### Beispiel 10

Technisches 2-Propylheptanal wurde in einer Rieselbettapparatur bei Temperatur 90°C und Druck 25*10⁵ Pa absolut an 3 g Katalysator in der Flüssigphase kontinuierlich hydriert. Es wurden stündlich 0,1 ml Edukt eingesetzt. Die Abgasmenge betrug 20 NL/h. Es wurde ein nach Beispiel 0 hergestellter, chromfreier Ni/Cu-Katalysator auf Al₂O₃-Trägermaterial verwendet. Allerdings wurde der Katalysator als Pulver eingesetzt, um Stofftransportproblemen zu begegnen. Skalierbarkeit auf technische Pellets war aber weiterhin gegeben. Der zeitliche Verlauf der Zusammensetzung des Produktgemisches ist in Tabelle 12 wiedergegen; die Zusammensetzung des Eduktgemisches entspricht dabei dem Tabelleneintrag zum Zeitpunkt t=0

**Tabelle 12: Zusammensetzung des Produktgemisches gemäß Beispiel 10**

| t / h | 2-Propylheptenal | 2-Propyl-2-heptenal | 2-Propylheptanol | Rest |
|---|---|---|---|---|
| 0 | 82,50 | 7,29 | 0,00 | 10,21 |
| 6 | 18,81 | 14,60 | 56,73 | 9,86 |
| 40 | 21,90 | 18,95 | 49,00 | 10,15 |
| 64 | 22,60 | 21,42 | 45,64 | 10,34 |
| 138 | 22,35 | 30,50 | 36,08 | 11,08 |
| 160,7 | 24,05 | 34,13 | 30,47 | 11,35 |
| 184 | 24,12 | 35,84 | 28,68 | 11,36 |

### Beispiel 11 (nicht erfindungsgemäß)

Der in Beispiel 10 beschriebene Versuch wurde analog mit demselben Edukt und unter denselben Bedingungen durchgeführt. Es wurde jedoch ein chromhaltiger Katalysator eingesetzt, wie er auch für die in DE19842370A1 beschrieben Versuche verwendet wurde. Der zeitliche Verlauf der Zusammensetzung des Produktgemisches ist in Tabelle 13 wiedergegen; die Zusammensetzung des Eduktgemisches entspricht dabei dem Tabelleneintrag zum Zeitpunkt t=0.

**Tabelle 13: Zusammensetzung des Produktgemisches gemäß Beispiel 11**

| t / h | 2-Propylheptenal | 2-Propyl-2-heptenal | 2-Propylheptanol | Rest |
|---|---|---|---|---|
| 0 | 82,50 | 7,29 | 0,00 | 10,21 |
| 6 | 26,05 | 16,89 | 46,90 | 10,16 |
| 40 | 28,85 | 24,56 | 36,06 | 10,53 |
| 64 | 29,01 | 26,24 | 34,21 | 10,54 |
| 138 | 26,81 | 32,89 | 29,20 | 11,10 |
| 160,7 | 24,29 | 39,88 | 23,97 | 11,86 |
| 184 | 23,78 | 41,55 | 22,81 | 11,86 |

### Fazit

Die Beispiele zeigen, dass der chromfrei hergestellte Katalysator geeignet ist, Oxo-Aldehyde zu hydrieren.

## Patentansprüche

1. Verfahren zur Herstellung von Alkoholen durch Hydrierung von Aldehyden, bei welchem mindestens ein Aldehyd sowie mindestens eine Begleitkomponente enthaltendes Einsatzgemisch in Gegenwart von Wasserstoff mit einem heterogenen Katalysator in Kontakt gebracht wird, wodurch ein Produktgemisch erhalten wird, welches zumindest den dem hydrierten Aldehyd entsprechenden Alkohol sowie zumindest ein Nebenprodukt enthält, wobei der Katalysator ein Trägermaterial sowie darauf aufgebracht Nickel und Kupfer umfasst,
**dadurch gekennzeichnet,**
**dass** der Katalysator in aktivierter Form die folgende, sich zu 100 Gew.-% ergänzende Zusammensetzung aufweist:
| | |
|---|---|
| Trägermaterial: | von 85 Gew.-% bis 95 Gew.-%; |
| Kupfer: | von 5.3 Gew.-% bis 8.4 Gew.-%; |
| Nickel: | von 2.2 Gew.-% bis 3.9 Gew.-%; |
| Chrom: | weniger als 50 Gew.-ppm; |
| Sonstige: | weniger als 1 Gew.-%, |
und dass es sich bei dem Trägermaterial um Aluminiumoxid oder um Silicumdioxid oder um ein Gemisch aus Aluminiumoxid und Siliciumdioxid handelt.

2. Verfahren nach Anspruch 1, **dadurch gekennzeichnet, dass** das spezifische Porenvolumen des Trägermaterial zwischen 0.5 ml/g bis 0.9 ml/g beträgt, bestimmt nach der Cyclohexan-Immersionsmethode, und dass die spezifische Oberfläche des Trägermaterials (BET-Oberfläche) zwischen 240 m²/g bis 280 m²/g beträgt, bestimmt nach der ISO-Methode 9277

3. Verfahren nach Anspruch 1 oder 2, **dadurch gekennzeichnet, dass** es bei einem Druck zwischen 15*10⁵ Pa und 25*10⁵ Pa und bei einer Temperatur zwischen 140°C und 180°C durchgeführt wird, wobei Druck und Temperatur so gewählt sind, dass Einsatzgemisch und Produktgemisch in einer flüssigen Phase vorliegen.

4. Verfahren nach Anspruch 3, **dadurch gekennzeichnet, dass** der Wasserstoff überstöchiometrisch gegenwärtig ist, wobei die Konzentration des Wasserstoffes so gewählt ist, dass zumindest ein Teil des Wasserstoffs in der flüssigen Phase gelöst vorliegt.

5. Verfahren nach Anspruch 1 oder nach einem der Ansprüche 2 bis 4, **dadurch gekennzeichnet, dass** das Einsatzgemisch aus einer Hydroformylierung stammt und als solches mehrere Aldehyde mit derselben Anzahl n an Kohlenstoffatomen sowie entsprechende Alkohole und Hochsieder enthält, wobei es sich bei n um eine natürliche Zahl zwischen drei und achtzehn handelt.

6. Verfahren nach Anspruch 5, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit fünf Kohlenstoffatomen: | 80 Gew.-% bis 100 Gew.-%; |
| Gesamtanteil der Alkohole mit fünf Kohlenstoffatomen: | 0 Gew.-% bis 1 Gew.-%; |
| Gesamtanteil an anderen Kohlenwasserstoffen: | 2 Gew.-% bis 20 Gew.-%. |

7. Verfahren nach Anspruch 5, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit neun Kohlenstoffatomen: | 25 Gew.-% bis 75 Gew.-%; |
| Gesamtanteil der Alkohole mit neun Kohlenstoffatomen: | 10 Gew.-% bis 55 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 3 Gew.-%. |

8. Verfahren nach Anspruch 5, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit neun Kohlenstoffatomen: | 15 Gew.-% bis 65 Gew.-%; |
| Gesamtanteil der Alkohole mit neun Kohlenstoffatomen: | 20 Gew.-% bis 65 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 1 Gew.-%. |

9. Verfahren nach Anspruch 5, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit zehn Kohlenstoffatomen: | 50 Gew.-% bis 100 Gew.-%; |
| Gesamtanteil der Alkohole mit zehn Kohlenstoffatomen: | 0 Gew.-% bis 40 Gew.-%; |
| Gesamtanteil weitere Kohlenwasserstoffe: | 0 Gew.-% bis 5 Gew.-%; |
| Anteil an Wasser: | 0.5 Gew.-% bis 5 Gew.-%. |

10. Verfahren nach Anspruch 5, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit dreizehn Kohlenstoffatomen: | 60 Gew.-% bis 85 Gew.-%; |
| Gesamtanteil der Alkohole mit dreizehn Kohlenstoffatomen: | 1 Gew.-% bis 20 Gew.-%; |
| Gesamtanteil weitere Kohlenwasserstoffe: | 10 Gew.-% bis 40 Gew.-%; |
| Anteil an Wasser: | 0.1 Gew.-% bis 1 Gew.-%. |

11. Verfahren nach Anspruch 1 oder nach einem der Ansprüche 2 bis 4, **dadurch gekennzeichnet, dass** das Einsatzgemisch aus mindestens zwei Hydroformylierungen stammt und als solches mehrere Aldehyde mit derselben Anzahl n an Kohlenstoffatomen und mehrere Aldehyde mit derselben Anzahl m an Kohlenstoffatomen, sowie jeweils entsprechende Alkohole und Hochsieder enthält, wobei es sich bei n und m um unterschiedliche natürliche Zahlen zwischen drei und achtzehn handelt.

## Claims

1. Process for the preparation of alcohols by hydrogenation of aldehydes, in which use mixture comprising at least one aldehyde and at least one accompanying component is brought into contact, in the presence of hydrogen, with a heterogeneous catalyst, giving a product mixture which comprises at least the alcohol corresponding to the hydrogenated aldehyde, and at least one by-product, where the catalyst comprises a support material, and nickel and copper applied thereto,
**characterized in that**
the catalyst in activated form has the following composition adding up to 100% by weight:
| | |
|---|---|
| support material: | from 85% by weight to 95% by weight; |
| copper: | from 5.3% by weight to 8.4% by weight; |
| nickel: | from 2.2% by weight to 3.9% by weight, |
| chromium: | less than 50 ppm by weight; |
| others: | less than 1% by weight, |
and **in that** the support material is aluminium oxide or silicon dioxide or a mixture of aluminium oxide and silicon dioxide.

2. Process according to Claim 1, **characterized in that** the specific pore volume of the support material is between 0.5 ml/g to 0.9 ml/g, determined by the cyclohexane immersion method, and that the specific surface area of the support material (BET surface area) is between 240 m²/g to 280 m²/g, determined by ISO method 9277.

3. Process according to Claim 1 or 2, **characterized in that** it is carried out at a pressure between 15*10⁵ Pa and 25*10⁵ Pa and at a temperature between 140°C and 180°C, the pressure and temperature being selected such that use mixture and product mixture are present in a liquid phase.

4. Process according to Claim 3, **characterized in that** the hydrogen is present in a superstoichiometric amount, the concentration of the hydrogen being selected such that at least some of the hydrogen is present dissolved in the liquid phase.

5. Process according to Claim 1 or according to one of Claims 2 to 4, **characterized in that** the use mixture originates from a hydroformylation and as such comprises a plurality of aldehydes with the same number n of carbon atoms, and corresponding alcohols and high boilers, where n is a natural number between three and eighteen.

6. Process according to Claim 5, **characterized in that** the use mixture has the following composition adding up to 100% by weight:
| | |
|---|---|
| total fraction of the aldehydes having five carbon 80% by weight to having atoms: | 80% by weight to 100% by weight; |
| total fraction of the alcohols having five carbon atoms: | 0% by weight to 1% by weight; |
| total fraction of other hydrocarbons: | 2% by weight to 20% by weight |

7. Process according to Claim 5, **characterized in that** the use mixture has the following composition adding up to 100% by weight:
| | |
|---|---|
| total fraction of the aldehydes having nine carbon atoms: | 25% by weight to 75% by weight; |
| total fraction of the alcohol shaving nine carbon atoms: | 10% by weight to 55% by weight; |
| total fraction of acetals; | 0.5% by weight to 5.5% by |
| total fraction of further hydrocarbons: | 0% by weight to 40% by weight; |
| water: | 0% by weight to 3% by weight. |

8. Process according to Claim 5, **characterized in that** the use mixture has the following composition adding up to 100% by weight:
| | |
|---|---|
| total fraction of the aldehydes having nine carbon atoms: | 15% by weight to 65% by weight; |
| total fraction of the alcohols having nine carbon atoms: | 20% by weight to 65% by weight; |
| total fraction of acetals: | 0.5% by weight to 5.5% by weight; |
| total fraction of further hydrocarbons: | 0% by weight to 40% by weight; |
| water: | 0% by weight to 1% by weight. |

9. Process according to Claim 5, **characterized in that** the use mixture has the following composition adding up to 100% by weight:
| | |
|---|---|
| total fraction of the aldehydes having ten carbon atoms: | 50% by weight to 100% by weight; |
| total fraction of the alcohols having ten carbon atoms: | 0% by weight to 40% by weight; |
| total fraction of further hydrocarbons: | 0% by weight to 5% by weight; |
| fraction of water: | 0.5% by weight to 5% by weight. |

10. Process according to Claim 5, **characterized in that** the use mixture has the following composition adding up to 100% by weight:
total fraction of the aldehydes having thirteen carbon atoms: 60% by weight to 85% by weight;
total fraction of the alcohols having thirteen carbon atoms: 1% by weight to 20% by weight;
total fraction of further hydrocarbons: 10% by weight to 40% by weight;
| | |
|---|---|
| fraction of water: | 0.1% by weight to 1% by weight. |

11. Process according to Claim 1 or according to one of Claims 2 to 4, **characterized in that** the use mixture originates from at least two hydroformylations and as such comprises a plurality of aldehydes having the same number n of carbon atoms and a plurality of aldehydes having the same number m of carbon atoms, and in each case corresponding alcohols and high boilers, where n and m are different natural numbers between three and eighteen.

## Revendications

1. Procédé de fabrication d'alcools par hydrogénation d'aldéhydes, selon lequel au moins un aldéhyde, ainsi qu'au moins un mélange de départ contenant un composant associé, sont mis en contact en présence d'hydrogène avec un catalyseur hétérogène, un mélange de produits étant ainsi obtenu, qui contient au moins l'alcool correspondant à l'aldéhyde hydrogéné, ainsi qu'au moins un produit secondaire, le catalyseur comprenant un matériau support, ainsi que du nickel et du cuivre appliqués sur celui-ci,
**caractérisé en ce que**
le catalyseur sous forme activée présente la composition suivante, dont la somme est de 100 % en poids :
| | |
|---|---|
| matériau support : | de 85 % en poids à 95 % en poids ; |
| cuivre : | de 5,3 % en poids à 8,4 % en poids ; |
| nickel : | de 2,2 % en poids à 3,9 % en poids ; |
| chrome : | moins de 50 ppm en poids ; |
| autres : | moins de 1 % en poids, |
et **en ce que** le matériau support consiste en de l'oxyde d'aluminium ou du dioxyde de silicium ou un mélange d'oxyde d'aluminium et de dioxyde de silicium.

2. Procédé selon la revendication 1, **caractérisé en ce que** le volume poreux spécifique du matériau support est compris entre 0,5 ml/g et 0,9 ml/g, déterminé par la méthode d'immersion dans du cyclohexane, et **en ce que** la surface spécifique du matériau support (surface BET) est comprise entre 240 m²/g et 280 m²/g, déterminée par la méthode ISO 9277.

3. Procédé selon la revendication 1 ou 2, **caractérisé en ce qu'**il est réalisé à une pression comprise entre 15*10⁵ Pa et 25*10⁵ Pa et à une température comprise entre 140 °C et 180 °C, la pression et la température étant choisies de sorte que le mélange de départ et le mélange de produits se présentent dans une phase liquide.

4. Procédé selon la revendication 3, **caractérisé en ce que** l'hydrogène est présent en une quantité sur-stoechiométrique, la concentration de l'hydrogène étant choisie de sorte qu'au moins une partie de l'hydrogène soit présent sous forme dissoute dans la phase liquide.

5. Procédé selon la revendication 1 ou selon l'une quelconque des revendications 2 à 4, **caractérisé en ce que** le mélange de départ provient d'une hydroformylation et contient en tant que tel plusieurs aldéhydes ayant le même nombre n d'atomes de carbone, ainsi que des alcools correspondants et des composants de point d'ébullition élevé, n étant un nombre naturel compris entre trois et dix-huit.

6. Procédé selon la revendication 5, **caractérisé en ce que** le mélange de départ présente la composition suivante, dont la somme est de 100 % en poids :
proportion totale des aldéhydes contenant cinq atomes de carbone : 80 % en poids à 100 % en poids ;
proportion totale des alcools contenant cinq atomes de carbone : 0 % en poids à 1 % en poids ;
proportion totale d'autres hydrocarbures : 2 % en poids à 20 % en poids.

7. Procédé selon la revendication 5, **caractérisé en ce que** le mélange de départ présente la composition suivante, dont la somme est de 100 % en poids :
proportion totale des aldéhydes contenant neuf atomes de carbone : 25 % en poids à 75 % en poids ;
proportion totale des alcools contenant neuf atomes de carbone : 10 % en poids à 55 % en poids ;
proportion totale d'acétals : 0,5 % en poids à 5,5 % en poids ;
proportion totale d'autres hydrocarbures : 0 % en poids à 40 % en poids ;
eau : 0 % en poids à 3 % en poids.

8. Procédé selon la revendication 5, **caractérisé en ce que** le mélange de départ présente la composition suivante, dont la somme est de 100 % en poids :
proportion totale des aldéhydes contenant neuf atomes de carbone : 15 % en poids à 65 % en poids ;
proportion totale des alcools contenant neuf atomes de carbone : 20 % en poids à 65 % en poids ;
proportion totale d'acétals : 0,5 % en poids à 5,5 % en poids ;
proportion totale d'autres hydrocarbures : 0 % en poids à 40 % en poids ;
eau : 0 % en poids à 1 % en poids.

9. Procédé selon la revendication 5, **caractérisé en ce que** le mélange de départ présente la composition suivante, dont la somme est de 100 % en poids :
proportion totale des aldéhydes contenant dix atomes de carbone : 50 % en poids à 100 % en poids ;
proportion totale des alcools contenant dix atomes de carbone : 0 % en poids à 40 % en poids ;
proportion totale d'autres hydrocarbures : 0 % en poids à 5 % en poids ;
proportion d'eau : 0,5 % en poids à 5 % en poids.

10. Procédé selon la revendication 5, **caractérisé en ce que** le mélange de départ présente la composition suivante, dont la somme est de 100 % en poids :
proportion totale des aldéhydes contenant treize atomes de carbone : 60 % en poids à 85 % en poids ;
proportion totale des alcools contenant treize atomes de carbone : 1 % en poids à 20 % en poids ;
proportion totale d'autres hydrocarbures : 10 % en poids à 40 % en poids ;
proportion d'eau : 0,1 % en poids à 1 % en poids.

11. Procédé selon la revendication 1 ou selon l'une quelconque des revendications 2 à 4, **caractérisé en ce que** le mélange de départ provient d'au moins deux hydroformylations et contient en tant que tel plusieurs aldéhydes ayant le même nombre n d'atomes de carbone et plusieurs aldéhydes ayant le même nombre m d'atomes de carbone, ainsi que des alcools correspondants à chaque fois et des composants de point d'ébullition élevé, n et m étant des nombres naturels différents compris entre trois et dix-huit.
